(19) Europäisches Patentamt
European Patent Office
Office européen des brevets

(11) **EP 2 716 222 A1**

(12) **EUROPEAN PATENT APPLICATION**
published in accordance with Art. 153(4) EPC

(43) Date of publication:
**09.04.2014 Bulletin 2014/15**

(51) Int Cl.:
**A61B 6/00** (2006.01)

(21) Application number: **12792577.4**

(22) Date of filing: **28.05.2012**

(86) International application number:
**PCT/JP2012/003459**

(87) International publication number:
**WO 2012/164900 (06.12.2012 Gazette 2012/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.05.2011 JP 2011120431**
**30.05.2011 JP 2011120432**
**30.05.2011 JP 2011120433**
**30.05.2011 JP 2011120439**

(71) Applicant: **FUJIFILM Corporation**
**Tokyo 106-8620 (JP)**

(72) Inventors:
• **NAKATSUGAWA, Haruyasu**
**Ashigarakami-gun**
**Kanagawa 258-8538 (JP)**

• **OHTA, Yasunori**
**Ashigarakami-gun**
**Kanagawa 258-8538 (JP)**
• **NISHINO, Naoyuki**
**Ashigarakami-gun**
**Kanagawa 258-8538 (JP)**
• **YOSHIDA, Yutaka**
**Ashigarakami-gun**
**Kanagawa 258-8538 (JP)**
• **KAMIYA, Takeshi**
**Ashigarakami-gun**
**Kanagawa 258-8538 (JP)**

(74) Representative: **Klunker . Schmitt-Nilson . Hirsch**
**Patentanwälte**
**Destouchesstrasse 68**
**80796 München (DE)**

(54) **METHOD AND DEVICE FOR ACQUIRING RADIATION EXPOSURE LEVEL, AND RADIOGRAPHIC IMAGING SYSTEM**

(57)    In the case where radiological imaging of a subject to be injected with a contrast agent is preformed continuously, obtaining a more accurate radiation exposure dose of the subject.

[Means for Solving the Problems]

When obtaining, based on a radiation image signal of each frame detected by a radiation image detector through continuous radiological imaging of a subject to be injected with a contrast agent, a radiation exposure dose of the subject due to the continuous radiological imaging, identifying a frame which includes an image signal representing an image of the contrast agent as a contrast agent frame from the radiation image signal of each frame, and correcting the radiation exposure dose based on information of the contrast agent frame and obtaining a radiation exposure dose of the subject for the radiological imaging of the contrast agent frame.

FIG.4

EP 2 716 222 A1

**Description**

Technical Field

The present invention relates to a radiation exposure dose obtaining method and apparatus, and a radiation image capturing system that obtain a radiation exposure dose of a subject to be injected with a contrast agent based on a radiation image signal of each frame obtained through continuous radiological imaging of the subject.

Background Art

Recently, it has been regarded as important to manage exposure doses of a patient due to radiological imaging in the medical front where radiological imaging is performed. As the relationship between the exposure dose of the patient due to radiological imaging and a disease such as cancer is drawing attention, it is necessary to maintain the exposure dose of a patient due to radiological imaging and to understand and manage the radiation exposure dose cumulatively received by the patient.

Consequently, for example, Patent Document 1 proposes that an exposure dose of a patient is obtained based on an image signal detected by a radiation image detector that detects a radiation image of the patient.

In the mean time, in the medical front where radiological imaging is performed, radiological imaging of moving pictures, such as fluoroscopic images, may be performed, as well as such radiological imaging of still images of patients. In the case of radiological imaging of such moving pictures, a large number of radiation images will be captured at a predetermined frame rate and the radiation exposure dose of the patient will be greater than that in the case where a still image is captured.

Consequently, the radiation exposure dose management of patients in radiological imaging of such moving pictures becomes more important.

[Prior Art Documents]

[Patent Documents]

Patent Document 1: Japanese Patent No. 4387644

Disclosure of the Invention

Patent Document 1 proposes that an exposure dose of a patient is obtained in the radiological imaging of a general still image, but proposes nothing about acquisition of a radiation exposure dose in the capturing of such fluoroscopic images described above.

In the radiological imaging of such fluoroscopic images, there may be a case where a contrast agent is injected, for example, into a blood vessel to obtain and observe a contrast agent image. In such a case, if the exposure dose of the patient is tried to be obtained based simply on the image signal of the radiation image detector as in Patent Document 1, there arises a concern that the calculated value may possibly differ from the actual exposure dose of the patient. That is, the radiation is absorbed by the contrast agent and the amount of radiation reached the radiation image detector is reduced.

Further, in the case where a contrast agent is injected during the radiological imaging of a fluoroscopic image, the contrast agent flows through a blood vessel or the like without remaining in one place, so that not all of the frames of the radiological imaging of the fluoroscopic image but some of them will include an image of the contrast agent. Therefore, it is necessary to obtain the exposure dose of the patient throughout the radiological imaging of the fluoroscopic image by taking into account the point described above.

In view of the circumstances described above, it is an object of the present invention to provide a radiation exposure dose obtaining method and apparatus, and a radiation image capturing system capable of obtaining a more accurate radiation exposure dose of a patient even in the case where radiological imaging of a subject to be injected with a contrast agent is performed continuously.

A radiation exposure dose obtaining apparatus of the present invention includes: a radiation exposure dose obtaining unit that obtains, based on a radiation image signal of each frame detected by a radiation image detector through continuous radiological imaging of a subject to be injected with a contrast agent, a radiation exposure dose of the subject due to the continuous radiological imaging; and a contrast agent frame identification unit that identifies a frame which includes an image signal representing an image of the contrast agent as a contrast agent frame from the radiation image signal of each frame, wherein the radiation exposure dose obtaining unit corrects the radiation exposure dose based on information of the contrast agent frame and obtains a radiation exposure dose of the subject for the radiological imaging of the contrast agent frame.

In the radiation exposure dose obtaining apparatus described above, the contrast agent frame identification unit may be configured to obtain information that indicates whether or not the contrast agent is being injected into the subject and to identify the contrast agent frame based on the obtained information.

Further, the contrast agent frame identification unit may be configured to obtain the information that indicates whether or not the contrast agent is being injected into the subject by obtaining information appended to the radiation image signal of each frame.

Still further, the information appended to the radiation image signal of each frame may be header information of the radiation image signal.

Further, the information that indicates whether or not the contrast agent is being injected into the subject may be based on a detection signal detected by a sensor provided in a contrast agent injection apparatus that injects the contrast agent into the subject.

Still further, the information that indicates whether or not the contrast agent is being injected into the subject may be information that indicates an injection start timing and an injection end timing of the contrast agent.

Further, the information that indicates an injection start timing and an injection end timing of the contrast agent may be an injection start time and an injection end time of the contrast agent into the subject.

Still further, the injection start time may be a drive start time of a contrast agent injection apparatus that injects the contrast agent into the subject and the injection end time may be a drive end time of the contrast agent injection apparatus.

A radiation image capturing system of the present invention includes the radiation exposure dose obtaining apparatus described above and a radiation image capturing apparatus that obtains the radiation image signal of each frame by performing the radiological imaging, wherein the radiation image capturing apparatus obtains information that indicates whether or not the contrast agent is being injected into the subject and stores the radiation image signal after appending the information to the radiation image signal.

The radiation image capturing system described above may include a contrast agent injection apparatus that injects the contrast agent to the subject and the image capturing apparatus is configured to obtain the information based on a detection signal detected by a sensor provided in the contrast agent injection apparatus.

Further, in the radiation image capturing system described above, the contrast agent frame identification unit may be configured to identify the contrast agent frame based on difference information which is based on the radiation image signals between predetermined frames of those of a plurality of frames detected through the continuous radiological imaging.

Still further, the contrast agent frame identification unit may be configured to identify the contrast agent frame based on a difference between a density histogram based on the radiation image signal of an $n^{th}$ frame (n is an integer greater than or equal to 2) of the radiation image signals of the plurality of frames and a density histogram based on the radiation image signal of a $(n-1)^{th}$ frame.

Further, the contrast agent frame identification unit may be configured to identify the contrast agent frame based on a difference in each corresponding pixel value between the radiation image signal of an $n^{th}$ frame (n is an integer greater than or equal to 2) of the radiation image signals of the plurality of frames and the radiation image signal of a $(n-1)^{th}$ frame.

Still further, the contrast agent frame identification unit may be configured to obtain dose information of radiation outputted from a radiation dose detection unit provided between a radiation source that emits the radiation to be applied to the subject and the subject, and to identify the contrast agent frame based on the obtained dose information and the radiation image signals of a plurality of frames detected through the continuous radiological imaging.

Further, the contrast agent frame identification unit may be configured to obtain a temporal variation in the dose information while the continuous radiological imaging is performed.

Still further, the contrast agent frame identification unit may be configured to calculate an index of dose of radiation received by the radiation image detector based on the radiation image signals of a plurality of frames detected through the continuous radiological imaging and to obtain a temporal variation in the index while the continuous radiological imaging is performed.

Further, the contrast agent frame identification unit may be configured to calculate a ratio between the dose information of radiation and the index calculated based on the radiation image signal of each frame, and to identify the contrast agent frame based on the ratio.

Still further, the radiation exposure dose obtaining unit may be configured to obtain a radiation exposure dose of the subject for the radiological imaging of the contrast agent frame based on the index calculated based on the radiation image signal of the contrast agent frame and a ratio between the dose information of radiation detected in the radiological imaging other than the contrast agent frame and the index calculated based on the radiation image signal other than the contrast agent frame.

Further, the radiation dose detection unit may be an area dosimeter that measures the radiation emitted from the radiation source.

Still further, the contrast agent frame identification unit may be configured to append information indicating as being a contrast agent frame to the frame identified as a contrast agent frame.

Further, the information indicating as being a contrast agent frame may be header information of the radiation image signal.

A radiation exposure dose obtaining method of the present invention obtains, based on a radiation image signal of each frame detected by a radiation image detector through continuous radiological imaging of a subject to be injected with a contrast agent, a radiation exposure dose of the subject due to the continuous radiological imaging, the method including the steps of: identifying a frame which includes an image signal representing an image of the contrast agent as a contrast agent frame from the radiation image signal of each frame; and correcting the radiation exposure dose based on information of the contrast agent frame and obtaining a radiation exposure dose of the subject for the radiological imaging of the contrast agent frame.

In the radiation exposure dose obtaining method described above, information that indicates whether or not the contrast agent is being injected into the subject may be obtained, and the contrast agent frame may be identified based on the obtained information.

Further, the contrast agent frame may be identified based on difference information which is based on radiation image signals between predetermined frames of those of a plurality of frames detected through the continuous radiological imaging.

Still further, dose information of radiation outputted from a radiation dose detection unit provided between a radiation source that emits the radiation to be applied to the subject and the subject, and the contrast agent frame may be identified based on the obtained dose information and the radiation image signals of a plurality of frames detected through the continuous radiological imaging.

According to the radiation exposure dose obtaining method and apparatus, and a radiation image capturing system of the present invention, when obtaining, based on a radiation image signal of each frame obtained through continuous radiological imaging of a subject to be injected with a contrast agent, a radiation exposure dose of the subject, a frame which includes an image signal representing an image of the contrast agent is identified as a contrast agent frame from the radiation image signal of each frame and the radiation exposure dose is corrected based on information of the contrast agent frame and a radiation exposure dose of the subject for the radiological imaging of the contrast agent frame is obtained. This allows a radiation exposure dose throughout the continuous radiological imaging to be obtained. In addition, a more accurate radiation exposure dose of the subject which takes into account the inclusion of an image of a contrast agent in the radiation image may be obtained.

In the radiation exposure dose obtaining method and apparatus, and a radiation image capturing system of the present invention described above, if an arrangement is adopted in which information that indicates whether or not the contrast agent is being injected into the subject is appended to the radiation image signal of each frame, and the contrast agent frame is identified based on the information, the contrast agent frame identification may be performed by a simpler method.

Further, if an arrangement is adopted in which the information that indicates whether or not the contrast agent is being injected into the subject is appended to the radiation image signal of each frame based on a detection signal detected by a sensor provided in a contrast agent injection apparatus that injects the contrast agent into the subject, the information to be appended to the radiation image signal may be obtained by a simple method.

Still further, if information that indicates an injection start timing and an injection end timing of the contrast agent is obtained and the contrast agent frame is identified based on the information, the contrast agent frame identification may be performed by a simple and inexpensive method without requiring any contrast agent injection detector, such as a sensor or the like.

According to the radiation exposure dose obtaining method and apparatus of the present invention, when obtaining, based on a radiation image signal of each frame obtained through continuous radiological imaging of a subject to be injected with a contrast agent, a radiation exposure dose of the subject, a frame that includes an image signal representing an image of the contrast agent is identified as a contrast agent frame based on difference information which is based on radiation image signals between predetermined frames of those of a plurality of frames detected through the continuous radiological imaging, and the radiation exposure dose described above is corrected based on the information of the identified contrast agent frame and a radiation exposure dose of the subject for the radiological imaging of the contrast agent frame is obtained. This allows a contrast agent frame to be identified appropriately by a simple calculation method. Further, a more accurate radiation exposure dose of the subject which takes into account the inclusion of an image of a contrast agent in the radiation image may be obtained.

According to the radiation exposure dose obtaining method and apparatus of the present invention, dose information of radiation outputted from a radiation dose detection unit provided between a radiation source that emits the radiation to be applied to the subject and the subject is obtained, then based on the obtained dose information and the radiation image signals of a plurality of frames detected through the continuous radiological imaging, a contrast agent frame which includes an image signal representing an image of a contrast agent is identified as a contrast agent frame from the plurality of frames, and the radiation exposure dose is corrected based on the information of the identified contrast agent frame and a radiation exposure dose of the subject for the radiological imaging of the contrast agent frame is obtained. This allows a contrast agent frame to be identified appropriately by a simple structure. Further, a radiation exposure dose throughout the radiological imaging which takes into account the inclusion of an image of a contrast agent in the radiation image may be obtained more accurately.

In the radiation exposure dose obtaining method and apparatus of the present invention described above, if an arrangement is adopted in which a temporal variation in the dose information while the continuous radiological imaging is performed is obtained and a temporal variation in index which is based on the image signals of a plurality of frames detected through the continuous radiological imaging, and a contrast agent frame is identified based on the ratio between the temporal change in the dose information and the temporal change in the index, a contrast agent frame may be identified by a simpler calculation method.

Further, if an arrangement is adopted in which a radiation exposure dose of the subject for the radiological imaging of the contrast agent frame is obtained based on the index calculated based on the radiation image signal of the contrast agent frame and a ratio between the dose information of radiation detected in the radiological imaging other than the contrast agent frame and the index calculated based on the radiation image signal other than the contrast agent frame, the radiation exposure dose for the radiological imaging of the contrast agent frame may be obtained by a simpler calculation method.

Still further, if a frame identified as a contrast agent frame is provided with appended information indicating as being a contrast agent frame, a contrast agent frame may be identified easily and instantaneously when obtaining a radiation exposure dose by taking into account the contrast agent frame.

Brief Description of Drawings

Figure 1 is a block diagram of a radiation image capturing system that uses a first embodiment of the radiation exposure dose obtaining apparatus of the present invention, illustrating an overall schematic configuration thereof.

Figure 2 illustrates, by way of example, a specific configuration of a contrast agent injection apparatus.

Figure 3 is a flowchart illustrating an operation of the radiation image capturing system that uses the first embodiment of the radiation exposure dose obtaining apparatus of the present invention.

Figure 4 shows a timing chart illustrating the relationship among radiation application timing, charge accumulation timing in the radiation image detector, and contrast agent detection signal, and a graph representing radiation exposure doses calculated based on the radiation image signal of each frame.

Figure 5 is a block diagram of a radiation image capturing system that uses a modification of the first embodiment the radiation exposure dose obtaining apparatus of the present invention, illustrating an overall schematic configuration thereof.

Figure 6 is a block diagram of a radiation image capturing system that uses a second embodiment of the radiation exposure dose obtaining apparatus of the present invention, illustrating an overall schematic configuration thereof.

Figure 7 is a flowchart illustrating an operation of the radiation image capturing system that uses the second embodiment of the radiation exposure dose obtaining apparatus of the present invention.

Figure 8 illustrates, by way of example, a density histogram of $n^{th}$ frame, a density histogram of $(n-1)^{th}$ frame, and a subtraction histogram of these.

Figure 9 illustrates frames f1 to f10 obtained by the imaging of a fluoroscopic image and a contrast agent injection start frame f3 and a contrast agent injection end frame f9 within the frames f1 to f10.

Figure 10 illustrates corresponding pixel values between a radiation image signal of $n^{th}$ frame and $(n-1)^{th}$ frame.

Figure 11 is a block diagram of a radiation image capturing system that uses a third embodiment of the radiation exposure dose obtaining apparatus of the present invention, illustrating an overall schematic configuration thereof.

Figure 12 illustrates an area dosimeter as an example of the radiation exposure dose detection unit.

Figure 13 is a flowchart illustrating an operation of the radiation image capturing system that uses the third embodiment of the radiation exposure dose obtaining apparatus of the present invention.

Figure 14 illustrates a method that identifies a contrast agent frame based on a temporal variation $G(t)$ in radiation dose detected by the radiation exposure dose detection unit and a temporal variation E.I. (t) in E.I. calculated based on the radiation image signal of each frame.

Figure 15 is a block diagram of a radiation image capturing system that uses a fourth embodiment of the radiation exposure dose obtaining apparatus of the present invention, illustrating an overall schematic configuration thereof.

Figure 16 illustrates a schematic configuration of the radiation image capturing apparatus shown in Figure 15.

Figure 17 illustrates a radiation image detector provided with first and second dose measurement sensors.

Figure 18 is a flowchart illustrating an operation of the radiation image capturing system that uses the fourth embodiment of the radiation exposure dose obtaining apparatus of the present invention.

Figure 19 illustrates, by way of example, temporal variations in radiation doses detected by the first and second dose measurement sensors.

Figure 20 illustrates a method of obtaining a radiation exposure dose when an artificial object frame is imaged.

Best Mode for Carrying Out the Invention

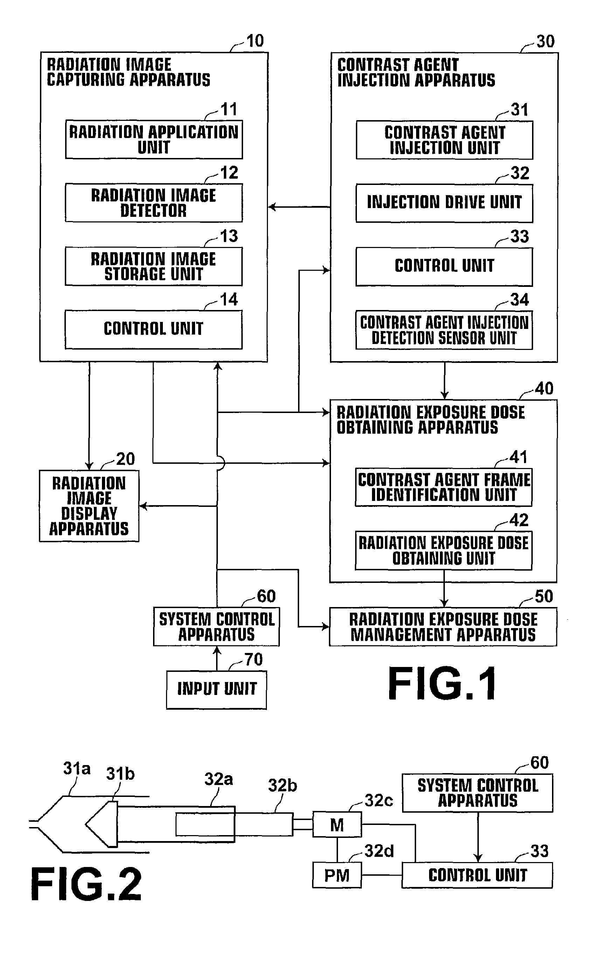

Hereinafter, a radiation image capturing system that uses a first embodiment of the radiation exposure dose obtaining apparatus of the present invention will be described with reference to the accompanying drawings. Figure 1 is a block diagram of the radiation image capturing system of the present embodiment, illustrating an overall schematic configuration thereof.

As illustrated in Figure 1, the radiation image capturing systemof the present embodiment includes a radiation image capturing apparatus 10 that captures a fluoroscopic image (moving picture) of a patient, a radiation image display apparatus 20 that displays the fluoroscopic image captured by the radiation image capturing apparatus 10, a contrast agent injection apparatus 30 that injects a contrast agent into a blood vessel or a lymph vessel of the patient, a radiation exposure dose obtaining apparatus 40 that obtains an exposure dose of the patient based on an image signal of the fluoroscopic image captured by the radiation image capturing apparatus 10, a radiation exposure dose management apparatus 50 that stores and manages the exposure dose of each patient obtained by the radiation exposure dose obtaining apparatus 40, and a system control apparatus 60 that performs overall control of the radiation image capturing system.

As illustrated in Figure 1, the radiation image capturing apparatus 10 includes a radiation application unit 11 that has an X-ray tube, an aperture stop, and the like and applies radiation emitted from the x-ray tube and passed through the aperture stop to a patient, a radiation image detector 12 that detects radiation transmitted through the patient and outputs a radiation image signal representing a radiation image of the patient, a radiation image storage unit 13 that stores the radiation image signal outputted from the radiation image detector 12, and a control unit 14 that performs overall control of the radiation image capturing apparatus 10.

The radiation image detector 12 allows repeated use for recording and reading of radiation images, and a so-called direct type radiation image detector that records a radiation image by generating and accumulating a charge by directly receiving radiation or a so-called indirect type radiation image detector that records a radiation image by converting radiation first to visible light and then converting the visible light to a charge and accumulating the charge may be used. As for the radiation image signal reading method for reading out the radiation image recorded by accumulating charges in the manner described above, a so-called TFT (thin film transistor) reading method in which a radiation image signal is read by ON/OFF switching thin film transistors and a so-called optical reading method in which a radiation image signal is read out by applying reading light are preferably used, but others may also be used.

The radiation image capturing apparatus 10 is used to capture a fluoroscopic image (moving picture) of a subject to be injected with a contrast agent by the contrast agent injection apparatus 30, and the control unit 14 controls the radiation application unit 11 and the radiation image detector 12 such that the fluoroscopic image is captured. More specifically, the control unit 14 controls the radiation application unit 11 to apply radiation at a predetermined frame rate and the radiation image detector 12 to perform recording and reading of a radiation image by the application of the radiation. Then, the radiation image signal of each frame outputted from the radiation image detector 12 is sequentially stored in the radiation image storage unit 13. Further, the control unit 14 of the present embodiment appends information indicating whether or not an image signal representing an image of the contrast agent is included in the radiation image signal of each frame when storing the radiation image signal of each frame in the radiation image storage unit 13, the operation of which will be described later in detail.

As for the structure of the radiation image capturing apparatus 10, a structure in which image capturing is performed with the patient being in the upright position or in the lateral position may be employed. Further, a structure that allows image capturing with the patient being both in upright position and lateral position may be employed.

The radiation image display apparatus 20 generates a display control signal by performing predetermined processing on the radiation image signal of each frame read out from the radiation image storage unit 13 of the radiation image capturing apparatus 10 and displays a fluoroscopic image of the patient on the monitor based on the display control signal.

The contrast agent injection apparatus 30 automatically injects a contrast agent into a patient based on an instruction input from the user or based on a preset injection start timing of the contrast agent. More specifically, the contrast agent injection apparatus 30 includes a contrast agent injection unit 31 formed of, for example, a syringe and injects a contrast agent into a blood vessel of the patient or the like, an injection drive unit 32 which drives the injection operation of the contrast agent injection unit 31, a control unit 33 that controls the operation of the injection drive unit 32, and a contrast agent injection detection sensor unit 34 that detects whether or not the contrast agent is being injected into a blood vessel or the like of the patient from the contrast agent injection apparatus 30.

As for the contrast agent injection unit 31, a contrast agent injection unit that includes, for example, a syringe 31a and a piston 31b provided in the syringe 10.

The injection drive unit 32 includes a plunger 32a to be connected to the piston 31b, a ball screw 32b to be connected to the plunger 32a, a motor 32c that rotates the ball screw 32b, and a potentiometer for detecting the position of the plunger 32a.

Then, when a contrast agent injection instruction is inputted at an input unit 60 to be described later, a control signal is outputted from the system control apparatus 60 to the control unit 33 of the contrast agent injection apparatus 30, and the control unit 33 outputs a drive voltage to the motor 32c in response to the inputted control signal. The rotation of the motor 32c is conveyed to the ball screw 32b and the plunger 32a is moved by the rotation of the ball screw 32b in a longitudinal direction of the syringe 31a, whereby the piston 31b is moved. Then a plunger position signal is outputted from the potentiometer 32d to the control unit 33 and the control unit controls the rotation of the motor 32c according to the feed back signal.

The contrast agent injection detection sensor unit 34 detects that a contrast agent is discharged from the contrast agent injection unit 31. More specifically, in the case where an input of instruction to start injection of a contrast agent is received at the input unit 60 as ON/OFF information of a switch, discharging of the contrast agent is detected by detecting the ON information while non-discharging of the contrast agent is detected by detecting the OFF information.

Further, in the case where the control is performed such that the contrast agent is discharged while the aforementioned switch is switched to ON and the contrast agent is not discharged while the switch is switched to OFF, the timing at which the switch is changed from OFF to ON may be detected as the contrast agent discharge start timing, then the time during which the switch is in ON state may be detected that the contrast agent discharge is in progress, and the timing at which the switch is changed from ON to OFF may be detected as the contrast agent discharge end timing.

Further, an arrangement may be adopted in which the drive power inputted to the motor 32c is detected by the contrast agent injection detection sensor 34 and the detection that the drive power is greater than or equal to a certain value is judged that the contrast agent discharge is in progress.

Still further, another arrangement may also be adopted in which a temporal change in the plunger position signal outputted from the potentiometer 32d is calculated and the detection that the amount of temporal change in the position of the plunger 32a is greater than or equal to a certain value is judged that the contrast agent discharge is in progress.

The contrast agent injection detection sensor unit 34 outputs the contrast agent detection signal to the control unit 14 of the radiation image capturing apparatus 10.

The radiation exposure dose obtaining apparatus 40 includes a contrast agent frame identification unit 41 that identifies a radiation image signal of a frame which includes an image signal of an image of a contrast agent as a contrast agent frame from radiation image signal of each frame read out from the radiation image storage unit 13 of the radiation image capturing apparatus 10, and a radiation exposure dose obtaining unit 42 that obtains a radiation exposure dose of the patient based on the radiation image signal of each frame read out from the radiation image storage unit 13 of the radiation image capturing apparatus 10. Operations of the contrast agent frame identification unit 41 and the radiation exposure dose obtaining unit 42 will be described later in detail.

The radiation exposure dose management apparatus 50 stores the radiation exposure dose obtained by the radiation exposure dose obtaining apparatus 40 and manages the radiation exposure dose of each patient.

The system control apparatus 60 outputs control signals to the radiation image capturing apparatus 10, the radiation image display apparatus 20, the contrast agent injection apparatus 30, the radiation exposure dose obtaining apparatus 40, and the radiation exposure dose management apparatus 50 to control the operations thereof and at the same time performs input/output signal control between these apparatuses. The system control apparatus 60 is provided with an input unit 70 which receives an instruction input and other inputs, such as image capturing conditions, patient ID information and the like, from the user. The input information received at the input unit 70 is outputted to each apparatus by the system control apparatus 60 as required.

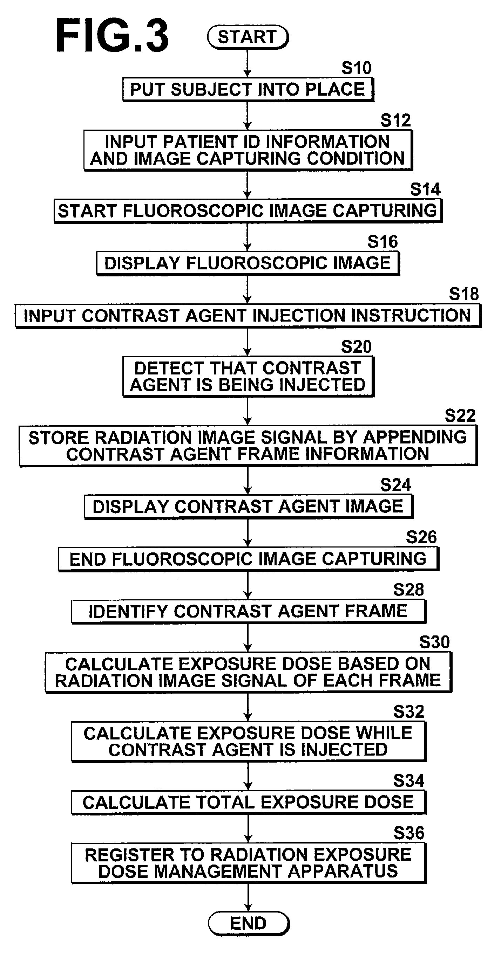

An operation of the radiation image capturing system of the present embodiment will now be described with reference to the flowchart of Figure 3.

First, a patient, the subject of the image capturing, is placed on an image capturing platform or the like provided in the radiation image capturing apparatus 10 and the patient is put into position (S10).

Then, ID information of the image capturing target patient and an image capturing condition are inputted by the user using the input unit 70 and the patient ID information is registered in the radiation exposure dose management apparatus 50 while the image capturing condition is set to the control unit 14 of the radiation image capturing apparatus 10 (S12). The image capturing condition may include a tube voltage, tube current, and application time in order to apply an appropriate dose of radiation to an image capturing region of the patient, as well as a frame rate for capturing a fluoroscopic image. As for the frame rate for capturing the fluoroscopic image, for example, a frame rate of 5fps to 60fps is set.

Next, an instruction to start fluoroscopic image capturing of the patient is inputted by the user using the input unit 70 and in response to the input, a control signal is outputted from the system control apparatus 60 to the radiation image capturing apparatus 10 to capture a fluoroscopic image, and the radiation image capturing apparatus 10 starts fluoroscopic image capturing according to the inputted control signal (S14).

More specifically, the X-ray tube of the radiation application unit 11 is controlled based on the inputted imaging condition and a predetermined dose of radiation is applied toward the patient intermittently at a predetermined frame rate.

Then, radiation transmitted through the patient is incident on the radiation image detector 12 and subjected to a photoelectrical conversion in the radiation image detector 12 and accumulated therein as a charge signal.

Thereafter, each time the application of radiation for each frame is ended, the charge signal accumulated in the radiation image detector 12 is read out by the control unit 14, then converted to a digital signal by an A/D converter (not shown), and stored in the radiation image storage unit 13.

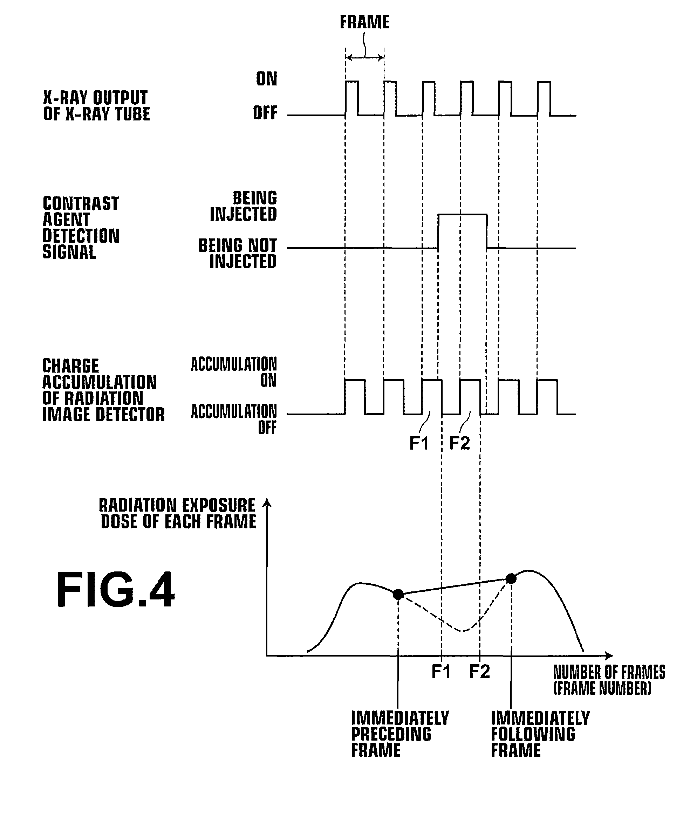

Figure 4 is a timing chart illustrating the application timing of radiation from the X-ray tube of the radiation application unit 11 and the charge accumulation timing of the radiation image detector 12. The period during which no charge accumulation is performed in the radiation image detector 12 (accumulation OFF period) is a period for reading out a charge signal from the radiation image detector 12.

The repeated performance of radiation application by the X-ray tube and the radiation image recording and reading in the radiation image detector 12 at a predetermined frame rate in the manner described above causes the radiation image signal of each frame to be sequentially stored in the radiation image storage unit 13.

Then, the radiation image signal of each frame stored in the radiation image storage apparatus 13 is sequentially read out and outputted to the radiation image display apparatus 20. The radiation image display apparatus 20 sequentially generates a display control signal based on the inputted radiation image signal of each frame and sequentially outputs the display control signal to the monitor to display a fluoroscopic image of the patient as a moving picture (S16). It is assumed here that the radiation image signal of each frame stored in the radiation image storage unit 13 will remain un-erased in the radiation image storage unit 13 after being read out.

Here, if the user desires to observe a contrast agent image, such as a blood vessel or the like, while the fluoroscopic image capturing and display are performed in the manner described above, a contrast agent injection instruction is inputted using the input unit 70 (S18).

When the contrast agent injection instruction is inputted at the input unit 70, a control signal is outputted from the system control apparatus 60 to the contrast agent injection apparatus 30. The contrast agent injection apparatus 30 starts contrast agent injection based on the inputted control signal. More specifically, the contrast agent injection unit 31 is driven by the injection drive unit 32 and a predetermined amount of contrast agent is discharged from the contrast agent injection unit 31.

Here, the contrast agent injection detection sensor unit 34 provided in the contrast agent injection apparatus 30 detects that the contrast agent is discharged and a detection signal is outputted to the control unit 14 of the radiation image capturing apparatus 10 (S20). When the contrast agent detection signal is inputted, the control unit 14 of the radiation image capturing apparatus 10 appends information indicating as having an image signal representing an image of the contrast agent (hereinafter, referred to as "contrast agent frame information")to the radiation image signal of a frame captured while the contrast agent detection signal is inputted as header information, and stores the radiation image signal in the radiation image storage unit 13 with the header information (S22). More specifically, if the contrast agent detection signal is inputted to the radiation image capturing apparatus 10 in the timing illustrated in Figure 4, the contrast agent frame information is appended to the radiation image signals of frames F1 and F2 captured while the contrast agent is injected and stored in the radiation image storage unit 13.

Then, the radiation image signal captured while the contrast agent is injected is also read out from the radiation image storage unit 13 and sequentially outputted to the radiation image display apparatus 20. The radiation image display apparatus 20 sequentially generates display control signals based on the inputted radiation image signals, and displays radiation images including a contrast agent image on the monitor based on the display control signals.

Then, after the discharge of the predetermined amount of contrast agent from the contrast agent injection apparatus 30 is completed and the contrast agent detection signal is not detected any more in the contrast agent injection detection sensor unit 34, radiation image signals captured are stored in the radiation image storage unit 13 without the contrast agent frame information being appended, and the radiation image signal of each frame is read out from the radiation image storage unit 13 in the manner described above and a fluoroscopic image is displayed on the radiation image display apparatus 20.

Thereafter, when an instruction to end the fluoroscopic image capturing is inputted by the user using the input unit 70, the system control apparatus 60 outputs a control signal to the radiation image capturing apparatus 10 to end the fluoroscopic image capturing, and the radiation image capturing apparatus 10 ends the fluoroscopic image capturing in response to the inputted control signal (S26).

When the fluoroscopic image capturing described above is ended, the system control apparatus 60 reads out radiation image signals of all frames stored in the radiation image storage unit 13 of the radiation image capturing apparatus 10 and inputs the radiation image signals to the radiation exposure dose obtaining apparatus 40.

Then, first, the contrast agent frame identification unit 41 identifies a radiation image signal of a frame to which contrast agent frame information is appended from the radiation image signals of all frames and identifies the frame as a contrast agent frame (S28).

Next, the radiation exposure dose obtaining unit 42 calculates a radiation exposure dose received by the patient during imaging of each frame of the fluoroscopic image based on the radiation image signal of each frame (S30). More specifically, an E.I. (Exposure Index) is calculated first based on the radiation image signal of each frame and the radiation exposure dose is obtained based on the E.I. in the present embodiment.

The E.I. is calculated in the following manner. First, a predetermined calculation area is set in a radiation image of each frame. The calculation area may be, for example, the entire area of the radiation image, an area arbitrarily set by the user, an area defined based on the imaged region information, or an area within 10% of the image size from the center of the radiation image. Otherwise, an area except for the so-called direct exposure area which may be obtained based on, for example, a histogram of the radiation image or an area of 90% of the entire density width from the central density of the radiation image may be employed. Alternatively, a specific calculation area may be set by combining the aforementioned conditions.

Next, a representative value V of the calculation area set in the above is calculated. As for the representative value V, the density value itself of the radiation image or a statistical characteristic value obtained by modifying the density value itself with the average value of the entire density values, median value, mode value, or trimmed mean value may be employed. Then, based on the representative value V, the E.I. of each frame is calculated by the formula given below:

$$\mathrm{E.I.} = C_0 \times g(V),$$

where:

  g(V): reverse calibration function; and
  $C_0$: 100·Gy (constant).

The g(V) is a function defined based on a radiation image obtained with the radiation quality of RQA5. The representative value V differs in magnitude due to difference in sensitivity arising from difference in scintillator used in the radiation image detector or difference in the setting method of the calculation area or the calculation method of the representative value V and g (V) is a function to normalize the differences. That is, if RQA5 radiation is received by any type of radiation image detector by the same radiation dose, the E.I. will become nearly the same value.

The radiation exposure dose received by the patient during imaging of each frame is calculated by the radiation exposure dose obtaining unit 42 using the E.I. calculated in the manner described above. As for the method of obtaining the radiation exposure dose using the E.I., for example, a function which defines these relationships or a lookup table may be set in advance.

The graph at the bottom of Figure 4 illustrates the radiation exposure dose of each frame connected by a line and the horizontal axis of the graph indicates the frame number from the start of imaging.

Here, in the case where a contrast agent is injected and a contrast agent image is captured in the middle of the fluoroscopic image capturing as described above, the radiation applied to the patient is absorbed by the contrast agent before reaching the radiation image detector 12. Therefore, if a radiation exposure dose is calculated based on the radiation image signal of the contrast agent frame, the calculated value will become smaller than that actually received by the patient. More specifically, if a radiation exposure dose is calculated based on the radiation image signals of frames F1, F2 illustrated in Figure 4, the calculated radiation exposure dose will become smaller than the actual value as illustrated by the dotted line in Figure 4.

Consequently, in the present embodiment, actual radiation exposure doses of the patient during the capturing of the contrast agent frames are obtained by obtaining radiation exposure doses corrected based on information of the contrast agent frames F1, F2 (S32). More specifically, linear interpolation is performed using radiation exposure doses calculated based on radiation image signals of the frames captured immediately before and after the frames F1, F2 which includes a contrast agent image, as illustrated in the graph of Figure 4, and radiation exposure doses at the imaging timing of the contrast agent frames F1, F2 on the linear line are obtained, whereby radiation exposure doses of the patient during the capturing of the contrast agent frames F1, F2 are obtained.

Next, the radiation exposure dose obtaining unit 42 calculates the total radiation exposure dose received by the patient by adding the radiation exposure doses during the capturing of the contrast agent frames obtained in the manner described above to the radiation exposure doses during the capturing of frames other than the contrast agent frames (S34).

The total radiation exposure dose obtained in the radiation exposure dose obtaining unit 42 in the manner described above is inputted to the radiation exposure dose management apparatus 50, and the radiation exposure dose management apparatus 50 registers the total radiation exposure dose with the ID information of the patient inputted in advance (S36). Then, the radiation exposure dose management apparatus 50 displays the registered total radiation exposure dose with the ID information of the patient as required or calculates a cumulative radiation exposure dose from the past for a particular patient and displays a warning message if the cumulative radiation exposure dose is greater than a predetermined specified value.

According to the radiation image capturing system of the present embodiment, a frame which includes an image signal representing an image of a contrast agent is identified as a contrast agent frame from the radiation image signal of each frame and the radiation exposure dose described above is corrected based on the information of the contrast agent frame and a radiation exposure dose of the subject during the radiological imaging of the contrast agent frame is obtained. This allows a radiation exposure dose throughout the continuous radiological imaging to be obtained and, at the same time, more accurate radiation exposure dose of a subject which takes into account the inclusion of an image of a contrast agent in the radiation image to be obtained.

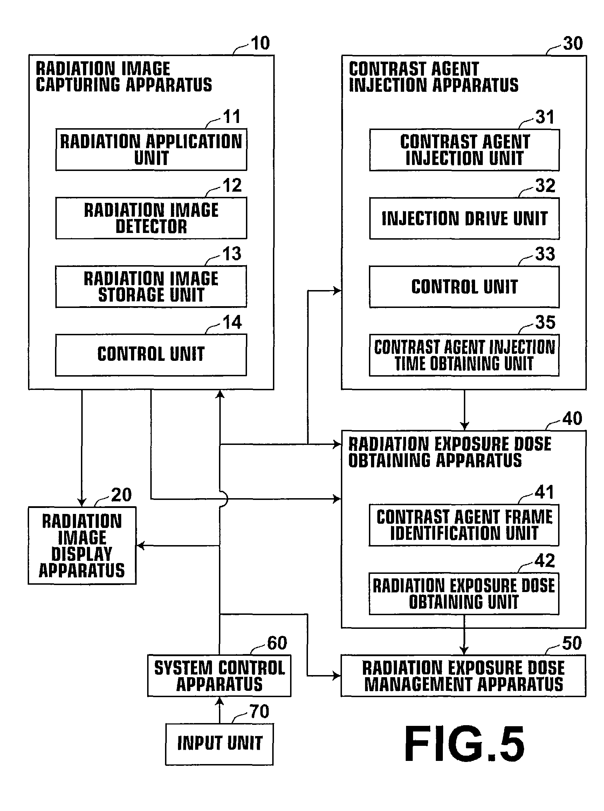

In the radiation image capturing system of the present embodiment described above, a contrast agent frame is identified after appending contrast agent frame information to a radiation image signal of a frame captured during a contrast agent detection signal is detected by the contrast agent injection detection sensor unit 34, but the method of identifying a contrast agent image is not limited to this. For example, a contrast agent injection time obtaining unit 35 may be provided, as illustrated in Figure 5, instead of the contrast agent injection detection sensor unit 34, and the contrast agent injection start and end times are obtained by the contrast agent injection time obtaining unit 35.

As for the contrast agent injection start and end times, for example, the drive start and end times of the contrast agent injection unit 31 by the inject drive unit 32 may be used or timings at which the instructions to start injection and to end injection are inputted by the user using the input unit 70 may be obtained.

Then, an arrangement may be adopted in which the contrast agent injection start and end times obtained by the contrast agent injection time obtaining unit 35, and imaging time at which each frame is captured (time at which a radiation image is recorded in the radiation image detector 12) in the radiation image capturing apparatus 10 are obtained by the contrast agent frame identification unit 41 and a frame captured between the contrast agent injection start time and the contrast agent injection end time is identified as a contrast agent frame.

Further, in the radiation image capturing system of the aforementioned embodiment, the radiation exposure dose is calculated after the fluoroscopic image capturing is completed, but not limited to this and the radiation exposure dose is calculated in the manner described above in the middle of the fluoroscopic image capturing and the radiation exposure dose of a subject in the middle of the imaging may be displayed or the like.

Further, in the radiation image capturing system described above, a radiation exposure dose of a patient during the capturing of a contrast agent frame is obtained through linear interpolation of radiation exposure doses calculated based on radiation image signals of immediately preceding and immediately following frames of the contrast agent frame. But the method is not limited to this and any other method may be used. For example, a radiation exposure dose calculated based on the radiation image signal of the immediately preceding frame or the immediately following frame may be employed directly as the radiation exposure dose while the contrast agent frame is captured, or a radiation exposure dose calculated based on the radiation image signal of another frame other than the contrast agent frame may be employed. Otherwise, a radiation exposure dose calculated based on the radiation image signal of a contrast agent frame may be corrected by adding a predetermined radiation exposure dose determined in advance or by multiplying it with a given coefficient which is greater than 1.

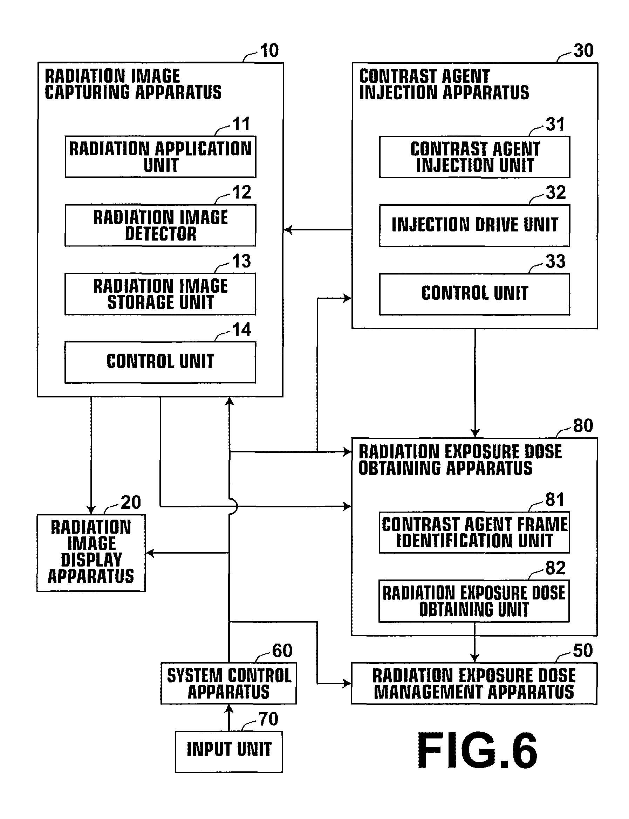

Next, a radiation image capturing system that uses a second embodiment of the radiation exposure dose obtaining apparatus of the present invention will be described. Figure 6 is a block diagram of the radiation image capturing system of the present embodiment, illustrating an overall schematic configuration thereof.

As illustrated in Figure 6, the radiation image capturing system of the present embodiment includes a radiation image capturing apparatus 10, a radiation image display apparatus 20, a contrast agent injection apparatus 30, a radiation exposure dose obtaining apparatus 80, a radiation exposure dose management apparatus 50, a system control apparatus 60, and an input unit 70.

The radiation image capturing apparatus 10, the radiation image display apparatus 20, the radiation exposure dose management apparatus 50, the system control apparatus 60, and the input unit 70 have identical configurations as those of the first embodiment. Further, the contrast agent injection apparatus 30 has a similar configuration to that of the first embodiment, except that it is not provided with the contrast agent injection detection senor unit 34.

In the radiation image capturing system of the present embodiment, the structure of the radiation exposure dose obtaining apparatus 80 is different from that of the first embodiment.

The radiation exposure dose obtaining apparatus 80 of the present embodiment includes a contrast agent frame identification unit 81 that identifies a radiation image signal of a frame which includes an image signal of an image of a contrast agent as a contrast agent frame from radiation image signal of each frame read out from the radiation image storage unit 13 of the radiation image capturing apparatus 10, and a radiation exposure dose obtaining unit 82 that obtains a radiation exposure dose of the patient based on the radiation image signal of each frame read out from the radiation image storage unit 13 of the radiation image capturing apparatus 10.

More specifically, the contrast agent frame identification unit 81 identifies a contrast agent frame from a plurality of frames by calculating a density histogram of the radiation image signal of each frame read out from the radiation image storage unit 13, subtracting a density histogram of $(n-1)^{th}$ frame (n is an integer greater than or equal to 2) from a density histogram of $n^{th}$ frame, and determining whether or not they are the frames capture during a contrast agent is injected based on the subtraction result.

Further, the contrast agent frame identification unit 81 of the present invention stores the radiation image signal of a frame determined to be a contrast agent frame after appending information indicating as being a contrast agent frame.

Operations of the contrast agent frame identification unit 81 and radiation exposure dose obtaining unit 82 will be described later in detail.

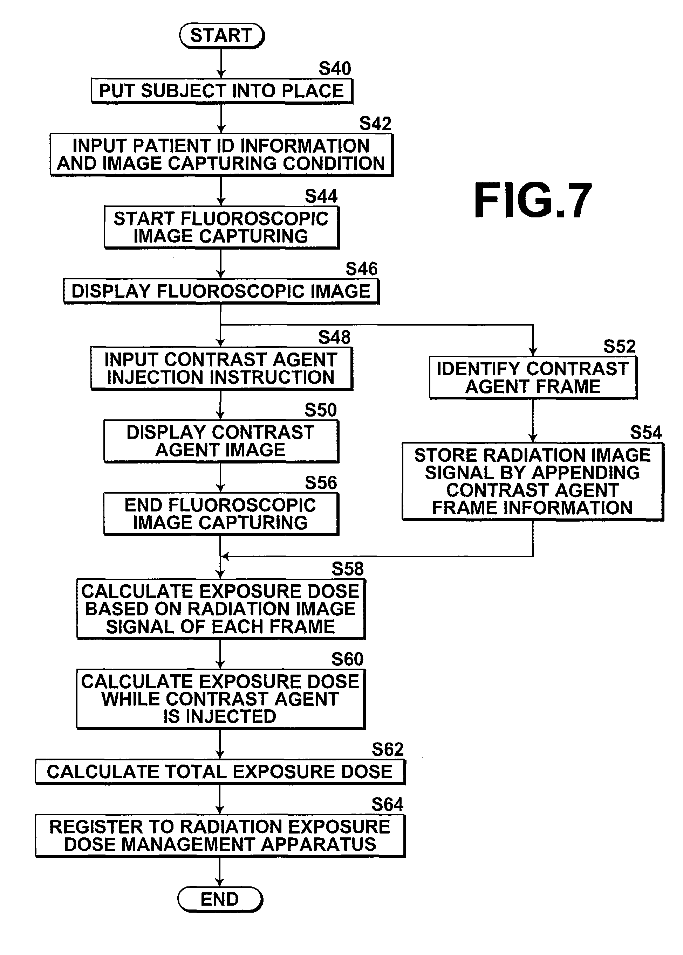

An operation of the radiation image capturing system of the present embodiment will now be described with reference to the flowchart of Figure 7.

First, a patient, the subject of the image capturing, is placed on an image capturing platform or the like provided in the radiation image capturing apparatus 10 and the patient is put into position (S40).

Then, ID information of the image capturing target patient and an image capturing condition are inputted by the user using the input unit 70 and the patient ID information is registered in the radiation exposure dose management apparatus 50 while the image capturing condition is set to the control unit 14 of the radiation image capturing apparatus 10 (S42). The image capturing condition may include a tube voltage, tube current, and application time in order to apply an appropriate dose of radiation to an image capturing region of the patient, as well as a frame rate for capturing a fluoroscopic image, as in the first embodiment. As for the frame rate for capturing the fluoroscopic image, for example, a frame rate of 5fps to 60fps is set.

Next, an instruction to start fluoroscopic image capturing of the patient is inputted by the user using the input unit 70 and in response to the input, a control signal is outputted from the system control apparatus 60 to the radiation image capturing apparatus 10 to capture a fluoroscopic image, and the radiation image capturing apparatus 10 starts fluoroscopic image capturing according to the inputted control signal (S44).

More specifically, the X-ray tube of the radiation application unit 11 is controlled based on the inputted imaging condition and a predetermined dose of radiation is applied toward the patient intermittently at a predetermined frame rate.

Then, radiation transmitted through the patient is incident on the radiation image detector 12 and subjected to a photoelectrical conversion in the radiation image detector 12 and accumulated therein as a charge signal.

Thereafter, each time the application of radiation for each frame is ended, the charge signal accumulated in the radiation image detector 12 is read out by the control unit 14, then converted to a digital signal by an A/D converter (not shown), and stored in the radiation image storage unit 13. Note that the application timing of radiation from the X-ray tube of the radiation application unit 11 and the charge accumulation timing of the radiation image detector 12 are identical to those in the timing chart of the first embodiment shown in Figure 4.

The repeated performance of radiation application by the X-ray tube and the radiation image recording and reading in the radiation image detector 12 at a predetermined frame rate in the manner described above causes the radiation image signal of each frame to be sequentially stored in the radiation image storage unit 13.

Then, the radiation image signal of each frame stored in the radiation image storage apparatus 13 is sequentially read out and outputted to the radiation image display apparatus 20. The radiation image display apparatus 20 sequentially generates a display control signal based on the inputted radiation image signal of each frame and sequentially outputs the display control signal to the monitor to display a fluoroscopic image of the patient as a moving picture (S46).

If the user desires to observe a contrast agent image, such as a blood vessel or the like, while the fluoroscopic image capturing and display are performed in the manner described above, a contrast agent injection instruction is inputted using the input unit 70 (S48).

When the contrast agent injection instruction is inputted at the input unit 70, a control signal is outputted from the system control apparatus 60 to the contrast agent injection apparatus 30. The contrast agent injection apparatus 30 start contrast agent injection based on the inputted control signal. More specifically, the contrast agent injection unit 31 is driven by the injection drive unit 32 and a predetermined amount of contrast agent is discharged from the contrast agent injection unit 31 and injected into a blood vessel or the like of the patient.

Then, the radiation image signal captured while the contrast agent is injected is also read out from the radiation image storage unit 13 and sequentially outputted to the radiation image display apparatus 20. The radiation image display apparatus 20 sequentially generates display control signals based on the inputted radiation image signals, and displays radiation images including a contrast agent image on the monitor based on the display control signals (S50).

The radiation image signals read out from the radiation image storage unit 13 of the radiation image capturing apparatus 10 are also sequentially inputted to the contrast agent frame identification unit 81 and the contrast agent frame identification unit 81 identifies a contrast agent frame which includes an image of the contrast agent based on the radiation image signal of each frame inputted sequentially (S52).

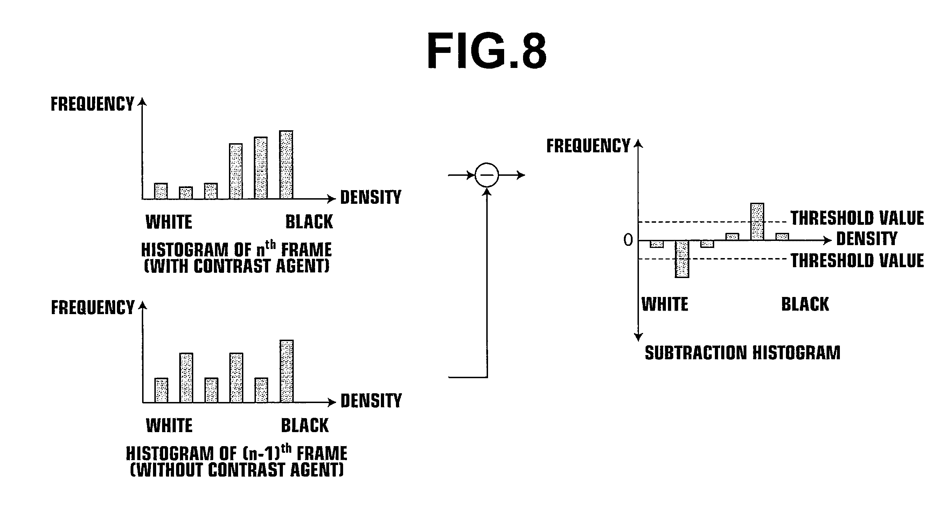

More specifically, as illustrated in Figure 8, a density histogram of the radiation image signal of $n^{th}$ frame (n is an integer greater than or equal to 2) and a density histogram of the radiation image signal of $(n-1)^{th}$ frame from the start of the fluoroscopic image capturing are sequentially calculated and a subtraction histogram is calculated by sequentially subtracting the density histogram of the $(n-1)^{th}$ frame from the density histogram of the $n^{th}$ frame. Then, as illustrated in Figure 8, if a density value whose frequency exceeds a preset threshold value appears in the frequency of each density value in the subtraction histogram, the $n^{th}$ frame is determined to be the contrast agent injection start frame. Note that the density histogram shown in Figure 8 is based on the assumption that the image signal of an image of the contrast agent is more on the black side than that of the surrounding area (smaller in signal value than the surround area), but conversely the density histogram may be based on the assumption that the image signal of an image of the contrast agent is more on the white side than that of the surrounding area (greater in signal value than the surround area), and, in that case, the subtraction histogram shown in Figure 8 becomes upside down.

Then, the frames following the contrast agent injection start frame until a contrast agent injection end frame, to be described later, appears are determined to be contrast agent frames. For the frames while the contrast agent is injected, a density value whose frequency exceeds the preset threshold value never appears in the subtraction histogram between $n^{th}$ frame and $(n-1)^{th}$ frame because $n^{th}$ frame and $(n+1)^{th}$ frame have substantially identical density histograms.

After the contrast agent injection start frame is determined, if a density value whose frequency exceeds the preset threshold value appears in a subtraction histogram between $n^{th}$ frame and $(n-1)^{th}$ frame again, the $n^{th}$ frame is determined to be a contrast agent injection end frame and frames following the contrast agent injection end frame are determined not to be contrast agent frames.

That is, the contrast agent frame identification unit 81 will eventually determines the frames from the contrast agent injection start frame to the frame immediately preceding the contrast agent injection end frame as contrast agent frames.

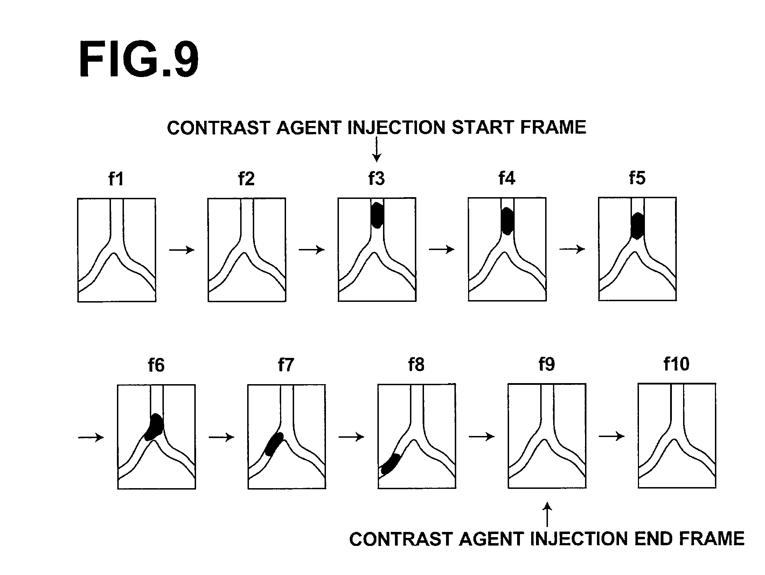

Figure 9 illustrates, in the case where frames f1 to f10 are imaged, a contrast agent injection start frame f3 and a contrast agent injection end frame f9 determined by the aforementioned determination method. In the example shown in Figure 9, the frames f3 to f8 are determined to be contrast agent frames.

Then, the contrast agent frame identification unit 81 appends information indicating as being a contrast agent frame (hereinafter, referred to as "contrast agent frame information")to the radiation image signal of the frame determined to be the contrast agent frame in the manner described above as header information, and stores the radiation image signal with the contrast agent frame information (S54).

Thereafter, when an instruction to end the fluoroscopic image capturing is inputted by the user using the input unit 70, the system control apparatus 60 outputs a control signal to the radiation image capturing apparatus 10 to end the fluoroscopic image capturing, and the radiation image capturing apparatus 10 ends the fluoroscopic image capturing in response to the inputted control signal (S56).

When the fluoroscopic image capturing described above is ended, a radiation exposure dose of the patient is obtained in the radiation exposure dose obtaining apparatus 80.

More specifically, the radiation image signal of each frame stored in the contrast agent frame identification unit 81 is outputted to the radiation exposure dose obtaining unit 82 with the contrast agent frame information.

Next, a radiation exposure dose received by the patient during imaging of each frame of the fluoroscopic image is calculated in the radiation exposure dose obtaining unit 82 based on the radiation image signal of each frame (S58) . More specifically, as in the first embodiment, an E.I. is calculated based on the radiation image signal of each frame and a radiation exposure dose is obtained based on the E.I. also in the present embodiment. The calculation method of E.I. and radiation exposure dose obtaining method base on the E.I. are as explained in the first embodiment.

Here, if a contrast agent is injected and a contrast agent image is captured in the middle of the fluoroscopic image capturing as described above, the radiation applied to the patient is absorbed by the contrast agent before reaching the radiation image detector 12. Therefore, if a radiation exposure dose is calculated based on the radiation image signal of the contrast agent frame, the calculated value will become smaller than that actually received by the patient.

Consequently, in the present embodiment, actual radiation exposure doses of the patient during the capturing of the contrast agent frames are obtained by obtaining radiation exposure doses corrected based on the information of contrast agent frames identified in the contrast agent frame identification unit 81 (S60) .

More specifically, as in the first embodiment, linear interpolation is performed using radiation exposure doses calculated based on radiation image signals of the frames captured immediately before and after the contrast agent frames (Figure 4) and radiation exposure doses at the imaging timing of the contrast agent frames on the linear line are obtained, whereby radiation exposure doses of the patient during the capturing of the contrast agent frames are obtained.

Next, the radiation exposure dose obtaining unit 82 calculates the total radiation exposure dose received by the patient by adding the radiation exposure doses during the capturing of the contrast agent frames obtained in the manner described above to the radiation exposure doses during the capturing of frames other than the contrast agent frames (S62).

The total radiation exposure dose obtained in the radiation exposure dose obtaining unit 82 in the manner described above is inputted to the radiation exposure dose management apparatus 50, and the radiation exposure dose management apparatus 50 registers the total radiation exposure dose with the ID information of the patient inputted in advance (S64). Then, the radiation exposure dose management apparatus 50 displays the registered total radiation exposure dose with the ID information of the patient as required or calculates a cumulative radiation exposure dose from the past for a particular patient and displays a warning message if the cumulative radiation exposure dose is greater than a predetermined specified value.

According to the radiation image capturing system of the present embodiment, a contrast agent frame is identified base on differential information which is based on radiation image signals between predetermined frames, and the radiation exposure dose is corrected based on the information of the contrast agent frame, whereby the radiation exposure dose of the subject during the radiological imaging of the contrast agent frame is obtained. This allows appropriate identification of a contrast agent frame to be performed by a simple calculation method. Further, a radiation exposure dose throughout the radiological imaging which takes into account the inclusion of an image of a contrast agent in the radiation image may be obtained more accurately.

In the embodiment described above, the density histogram of (n-1)th frame is subtracted from the density histogram of nth frame, but the subtracted density histogram is not necessary the density frame of (n-1)th frame and, for example, the density histogram of any one frame of those already determined not to be contrast agent frames may be used. Otherwise, an average density histogram of any two or more frames of those already determined not to be contrast agent frames may be used.

Further, in the embodiment described above, if the exposure dose of radiation applied by the radiation application unit 11, the density histogram will have an offset in a density axis direction or the density histogram will be enlarged or reduced. Therefore, it is desirable that the density histogram is normalized in the density axis direction. The normalization method is substantially identical to the density equalization technique between two or more images in subtraction of radiation images and is a known method so that it will not be elaborated upon further here.

Still further, in the embodiment described above, the contrast agent injection start frame and the contrast agent injection end frame are identified using the density histogram of the radiation image signal of each frame, but the identification method is not limited to this and other methods may be used. Hereinafter, another method for identifying the contrast agent injection start frame and the contrast agent injection end frame will be described.

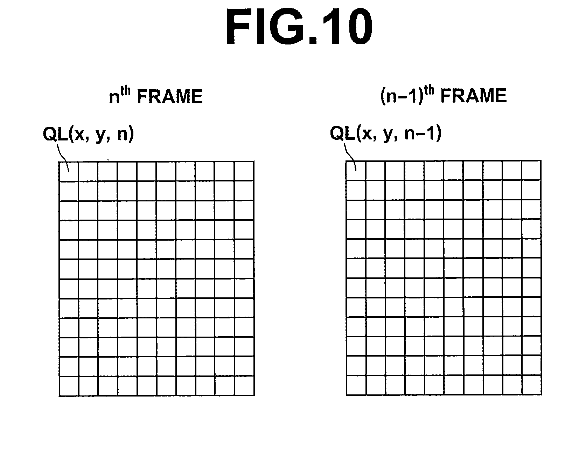

First, as illustrated in Figure 10, when a pixel value of the radiation image signal of nth frame at a pixel position (x, y) on the radiation image detector 12 is taken as QL(x, y, n) and a pixel value of the radiation image signal of (n-1)th frame at a pixel position (x, y) on the radiation image detector 12 is taken as QL(x, y, n-1), D (x, y, n) is obtained for all pixels by calculating the formula given below:

$$D(x, y, n) = QL(x, y, (n-1)) - QL(x, y, n).$$

Then, D(x, y, n) is sequentially calculated for each frame and if the total number of pixels in which the D(x, y, n) is greater than a first threshold value a (a>0) exceeds a second threshold value c, the nth frame at that time is determined to be the contrast agent injection start frame.

Then, after the determination of the contrast agent injection start frame is made, if the total number of pixels in which the D(x, y, n) is smaller than a third threshold value a (b<0) exceeds a fourth threshold value d, the nth frame at that time is determined to be the contrast agent injection end frame.

Use of the method described above may identify the contrast agent injection start frame and the contrast agent injection end frame. Note that the aforementioned determination method is based on the assumption that the image signal of an image of the contrast agent is more on the black side than that of the surrounding area (smaller in signal value than the surround area), but conversely the density histogram may be based on the assumption that the image signal of an image of the contrast agent is more on the white side than that of the surrounding area (greater in signal value than the surround area), and, in that case, the contrast agent injection start frame determination method and the contrast agent injection end frame determination method are reversed.

In the aforementioned determination method, the contrast agent injection start frame and the contrast agent injection end frame are determined by subtracting a pixel value of nth frame from a pixel value of (n-1)th frame, but not limited to this and, for example, a pixel value of nth frame may be subtracted from a pixel value of any one frame of those already determined not to be contrast agent frames. Otherwise, the pixel value of nth frame may be subtracted from an average pixel value of any two or more frames already determined not to be contrast agent frames.

Further, in the radiation image capturing system of the second embodiment described above, the radiation exposure dose is calculated after the fluoroscopic image capturing is completed, but not limited to this and the radiation exposure dose is calculated in the manner described above in the middle of the fluoroscopic image capturing and the radiation exposure dose of a subject in the middle of the imaging may be displayed or the like, as in the first embodiment.

Still further, in the radiation image capturing system of the second embodiment described above, a radiation exposure dose of a patient during the capturing of a contrast agent frame is obtained through linear interpolation of radiation exposure doses calculated based on radiation image signals of immediately preceding and immediately following frames of the contrast agent frame. But the method is not limited to this and, for example, a radiation exposure dose calculated based on the radiation image signal of the immediately preceding frame or the immediately following frame may be employed directly as the radiation exposure dose during the capturing of the contrast agent frame, or a radiation exposure dose calculated based on the radiation image signal of another frame other than the contrast agent frame may be employed as in the first embodiment. Otherwise, the radiation exposure dose calculated based on the radiation image signal of the contrast agent frame may be corrected by adding a predetermined radiation exposure dose determined in advance or by multiplying it with a preset constant which is greater than 1.

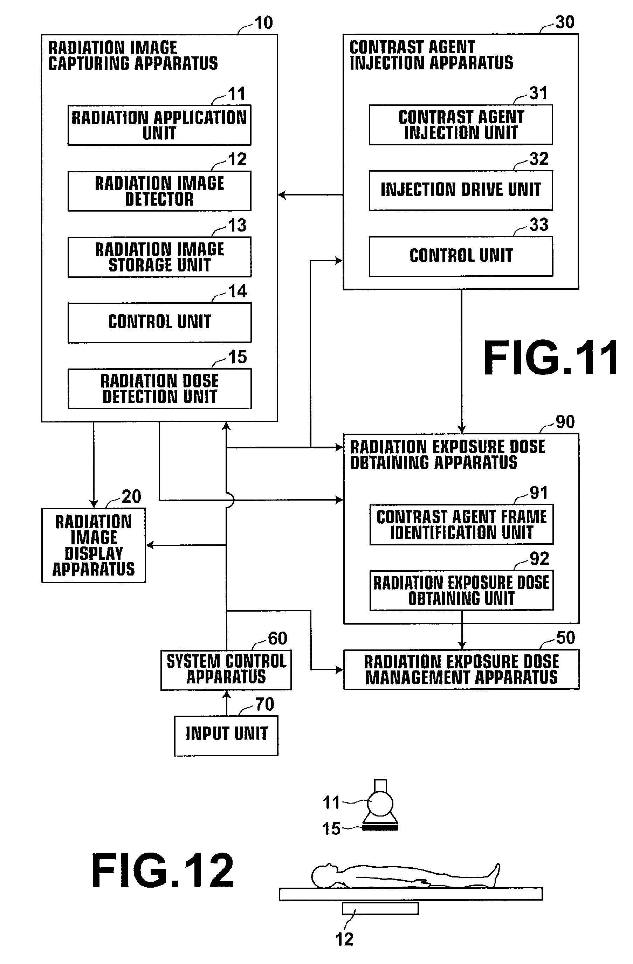

Next, a radiation image capturing system that uses a third embodiment of the radiation exposure dose obtaining apparatus of the present invention will be described. Figure 11 is a block diagram of the radiation image capturing system of the present embodiment, illustrating an overall schematic configuration thereof.

As illustrated in Figure 11, the radiation image capturing system of the present embodiment includes a radiation image capturing apparatus 10, a radiation image display apparatus 20, a contrast agent injection apparatus 30, a radiation exposure dose obtaining apparatus 90, a radiation exposure dose management apparatus 50, a system control apparatus 60, and an input unit 70.

The radiation image display apparatus 20, the radiation exposure dose management apparatus 50, the system control apparatus 60, and the input unit 70 have identical configurations as those of the first embodiment. Further, the contrast agent injection apparatus 30 has a similar configuration to that of the first embodiment, except that it is not provided with the contrast agent injection detection senor unit 34.

In the radiation image capturing system of the present embodiment, the structures of the radiation image capturing apparatus 10 and the radiation exposure dose obtaining apparatus 80 are different from those of the first embodiment.

The radiation image capturing apparatus 10 of the present embodiment further includes a radiation dose detection unit 15 in addition to the structure of the first embodiment. The other configuration is identical to that of the radiation image capturing apparatus 10 of the first embodiment.

The radiation dose detection unit 15 is provided between the radiation application unit 11 and a patient and detects the dose of radiation outputted from the radiation application unit 11 and before reaching the patient. As for the radiation dose detection unit 15, for example, an area dosimeter provided at the radiation output opening of the radiation application unit 11, like that shown in Figure 12, may be used.

The radiation dose detection unit 15 sequentially outputs information of the detected dose of radiation to a contrast agent identification unit 91, to be described later, in parallel with fluoroscopic image capturing.

As for the structure of the radiation image capturing apparatus 10, a structure in which image capturing is performed with the patient being in the lateral position, as shown in Figure 12, or in the upright position may be employed. Further, a structure that allows image capturing with the patient being both in upright position and lateral position may be employed.

The radiation exposure dose obtaining apparatus 90 of the present embodiment includes a contrast agent frame identification unit 91 that identifies a radiation image signal of a frame which includes an image signal of an image of a contrast agent as a contrast agent frame from the radiation image signal of each frame read out from the radiation image storage unit 13 of the radiation image capturing apparatus 10, and a radiation exposure dose obtaining unit 92 that obtains a radiation exposure dose of the patient based on the radiation image signal of each frame read out from the radiation image storage unit 13 of the radiation image capturing apparatus 10.

The contrast agent frame identification unit 91 identifies a contrast agent frame from a plurality of frames obtained by the fluoroscopic image capturing based on information of radiation dose outputted from the radiation dose detection unit 15 and the radiation image signal of each frame read out from the radiation image storage unit 13.

Further, the contrast agent frame identification unit 91 appends information indicating as being a contrast agent frame to the frame determined to be a contrast agent frame.

Operations of the contrast agent frame identification unit 91 and the radiation exposure dose obtaining unit 92 will be described later in detail.

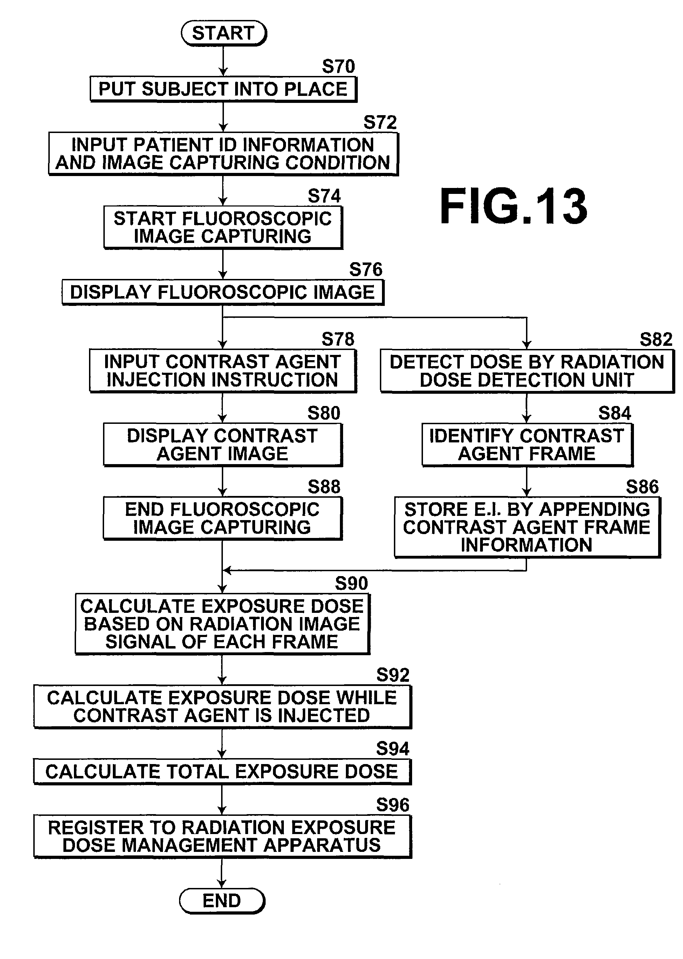

An operation of the radiation image capturing system of the present embodiment will now be described with reference to the flowchart of Figure 13.

First, a patient, the subject of the image capturing, is placed on an image capturing platform or the like provided in the radiation image capturing apparatus 10 and the patient is put into position (S70).

Then, ID information of the image capturing target patient and an image capturing condition are inputted by the user using the input unit 70 and the patient ID information is registered in the radiation exposure dose management apparatus 50 while the image capturing condition is set to the control unit 14 of the radiation image capturing apparatus 10 (S72). The image capturing condition may include a tube voltage, tube current, and application time in order to apply an appropriate dose of radiation to an image capturing region of the patient, as well as a frame rate for capturing a fluoroscopic image, as in the first embodiment. As for the frame rate for capturing the fluoroscopic image, for example, a frame rate of 5fps to 60fps is set.

Next, an instruction to start fluoroscopic image capturing of the patient is inputted by the user using the input unit 70 and in response to the input, a control signal is outputted from the system control apparatus 60 to the radiation image capturing apparatus 10 to capture a fluoroscopic image, and the radiation image capturing apparatus 10 starts fluoroscopic image capturing according to the inputted control signal (S74).

More specifically, the X-ray tube of the radiation application unit 11 is controlled based on the inputted imaging condition and a predetermined dose of radiation is applied toward the patient intermittently at a predetermined frame rate.

Then, radiation transmitted through the patient is incident on the radiation image detector 12 and subjected to a photoelectrical conversion in the radiation image detector 12 and accumulated therein as a charge signal.

Thereafter, each time the application of radiation for each frame is ended, the charge signal accumulated in the radiation image detector 12 is read out by the control unit 14, then converted to a digital signal by an A/D converter (not shown), and stored in the radiation image storage unit 13. Note that the application timing of radiation from the X-ray tube of the radiation application unit 11 and the charge accumulation timing of the radiation image detector 12 are identical to those in the timing chart of the first embodiment shown in Figure 4.

The repeated performance of radiation application by the X-ray tube and the radiation image recording and reading in the radiation image detector 12 at a predetermined frame rate in the manner described above causes the radiation image signal of each frame to be sequentially stored in the radiation image storage unit 13.

Then, the radiation image signal of each frame stored in the radiation image storage apparatus 13 is sequentially read out and outputted to the radiation image display apparatus 20. The radiation image display apparatus 20 sequentially generates a display control signal based on the inputted radiation image signal of each frame and sequentially outputs the display control signal to the monitor to display a fluoroscopic image of the patient as a moving picture (S76).

If the user desires to observe a contrast agent image, such as a blood vessel or the like, while the fluoroscopic image capturing and display are performed in the manner described above, a contrast agent injection instruction is inputted using the input unit 70 (S78).

When the contrast agent injection instruction is inputted at the input unit 70, a control signal is outputted from the system control apparatus 60 to the contrast agent injection apparatus 30. The contrast agent injection apparatus 30 starts contrast agent injection based on the inputted control signal. More specifically, the contrast agent injection unit 31 is driven by the injection drive unit 32 and a predetermined amount of contrast agent is discharged from the contrast agent injection unit 31 and injected into a blood vessel or the like of the patient.

Then, the radiation image signal captured while the contrast agent is injected is also read out from the radiation image storage unit 13 and sequentially outputted to the radiation image display apparatus 20. The radiation image display apparatus 20 sequentially generates display control signals based on the inputted radiation image signals, and displays radiation images including a contrast agent image on the monitor based on the display control signals (S80) .

Here, the dose of radiation outputted from the radiation application unit 11 is sequentially detected by the radiation dose detection unit 15 in parallel with the radiation image capturing and display described above, and the detected radiation dose is sequentially inputted to the contrast agent frame identification unit 91 of the radiation exposure dose obtaining apparatus 90 (S82). Further, the radiation image signal sequentially read out from the radiation image storage unit 13 is inputted also to the contrast agent frame identification unit 91. The contrast agent frame identification unit 91 identifies a contrast agent frame in which an image of the contrast agent is imaged based on the temporal variation in the dose of inputted radiation and the temporal variation in the inputted radiation image signal of each frame (S84) .

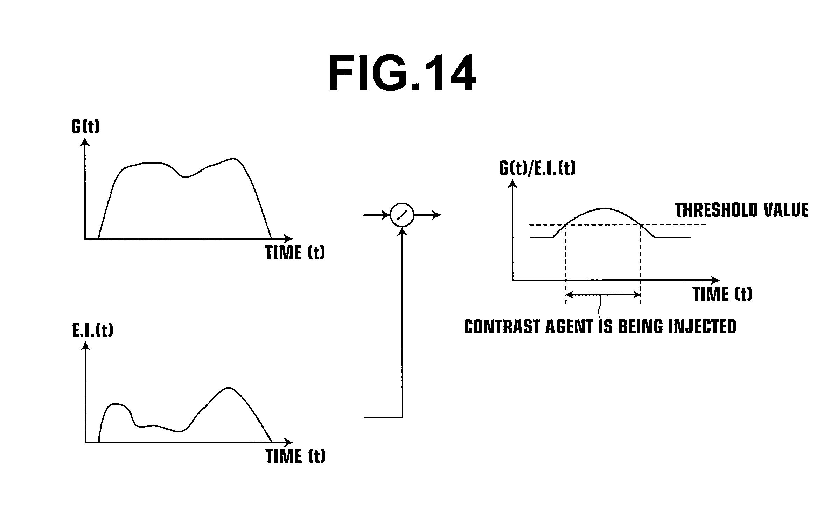

More specifically, the contrast agent frame identification unit 91 obtains a temporal variation $G(t)$ in the dose of radiation, as illustrated in Figure 14, based on the dose of inputted radiation, and calculates an E.I. based on the inputted radiation image signal of each frame and obtains a temporal variation E.I. (t) of E.I., as illustrated in Figure 14.

Here, the E.I. serves as an index of the dose of radiation received by the radiation image detector 12 through a patient, the calculation method of which is as described in the first embodiment.

As illustrated in Figure 14, the contrast agent identification unit 91 calculates a temporal variation in the divided value $G(t)/E.I.(t)$ between the $G(t)$ and E.I.(t) obtained in the manner described above. It is assumed here that the contrast agent frame identification unit 91 also obtains the detection timing of the dose of radiation in the radiation dose detection unit 15 and the detection timing of radiation image signal of each frame in the radiation image detector 12, and the time axes of the $G(t)$ and the E.I. (t) correspond to each other.

Then, the contrast agent frame identification unit 91 compares the value of $G(t)/E.I.(t)$ with a preset value and if the value of $G(t)/E.I.(t)$ is greater than or equal to the threshold value, the contrast agent frame identification unit 91 determines the frame obtained at the time t corresponding to the value as a contrast agent frame.

Then, the contrast agent frame identification unit 91 appends information indicating as being a contrast agent frame (hereinafter, referred to as "contrast agent frame information") to the E.I. calculated based on the radiation image signal of the frame identified as a contrast agent frame in the manner described above and stores the E.I. with the contrast agent frame information (S86).

Thereafter, when an instruction to end the fluoroscopic image capturing is inputted by the user using the input unit 70, the system control apparatus 60 outputs a control signal to the radiation image capturing apparatus 10 to end the fluoroscopic image capturing, and the radiation image capturing apparatus 10 ends the fluoroscopic image capturing in response to the inputted control signal (S88).

When the fluoroscopic image capturing described above is ended, a radiation exposure dose of the patient is obtained in the radiation exposure dose obtaining apparatus 90.

More specifically, the E.I. corresponding to each frame stored in the contrast agent frame identification unit 91 is outputted to the radiation exposure dose obtaining unit 92 with the contrast agent frame information first.

Then, a radiation exposure dose received by the patient during imaging of each frame of the fluoroscopic image is calculated in the radiation exposure dose obtaining unit 92 based on the E.I. corresponding to each frame (S90) . Note that the radiation exposure dose obtaining method using the E.I. is as explained in the first embodiment.

Here, if a contrast agent is injected and a contrast agent image is captured in the middle of the fluoroscopic image capturing as described above, the radiation applied to the patient is absorbed by the contrast agent before reaching the radiation image detector 12. Therefore, if a radiation exposure dose is calculated using the E.I. based on the radiation image signal of the contrast agent frame, the calculated value will become smaller than that actually received by the patient.

Consequently, in the present embodiment, actual radiation exposure doses of the patient during the capturing of the contrast agent frames are obtained by obtaining radiation exposure doses corrected based on the information of contrast agent frames identified in the contrast agent frame identification unit 91 (S92) .

More specifically, linear interpolation is performed using radiation exposure doses calculated based on radiation image signals of the frames captured immediately before and after the contrast agent frames (Figure 4) and radiation exposure doses at the imaging timing of the contrast agent frames on the linear line are obtained, whereby radiation exposure doses of the patient during the capturing of the contrast agent frames are obtained.

Then, the radiation exposure dose obtaining unit 92 calculates the total radiation exposure dose received by the patient by adding the radiation exposure doses during the capturing of the contrast agent frames obtained in the manner described above to the radiation exposure doses during the capturing of frames other than the contrast agent frames (S94).

The total radiation exposure dose obtained in the radiation exposure dose obtaining unit 92 in the manner described above is inputted to the radiation exposure dose management apparatus 50, and the radiation exposure dose management apparatus 50 registers the total radiation exposure dose with the ID information of the patient inputted in advance (S96). Then, the radiation exposure dose management apparatus 50 displays the registered total radiation exposure dose with the ID information of the patient as required or calculates a cumulative radiation exposure dose from the past for a particular patient and displays a warning message if the cumulative radiation exposure dose is greater than a predetermined specified value.

According to the radiation image capturing system of the present embodiment, a contrast agent frame is identified based on the information of the dose of radiation outputted from the radiation dose detection unit provided between the radiation source and the subject and radiation image signals of a plurality of frames detected through the continuous radiological imaging, and the radiation exposure dose is corrected based on the information of the identified contrast agent frame, whereby the radiation exposure dose of the subject during the radiological imaging of the contrast agent frame is obtained. This allows appropriate identification of a contrast agent frame to be made by a simple structure. Further, a radiation exposure dose throughout the radiological imaging which takes into account the inclusion of an image of a contrast agent in the radiation image may be obtained more accurately.

In the radiation image capturing system of the embodiment described above, the radiation exposure dose is calculated after the fluoroscopic image capturing is completed, but not limited to this and the radiation exposure dose is calculated in the manner described above in the middle of the fluoroscopic image capturing and the radiation exposure dose of a subject in the middle of the imaging may be displayed or the like, as in the first embodiment.

Further, in the radiation image capturing system of the embodiment described above, a radiation exposure dose of a patient during the capturing of a contrast agent frame is obtained through linear interpolation of radiation exposure doses calculated based on the radiation image signals of immediately preceding and immediately following frames of the contrast agent frame. But other methods may also be employed for obtaining the radiation image exposure dose corresponding to a contrast agent frame.

More specifically, for example, when an E.I. calculated based on the radiation image signal of a contrast agent frame is taken as $E.I.(t_k)$, and $G(t)/E.I.(t)$ corresponding to a frame other than a contrast agent frame is taken as $G(t_m)/E.I.(t_m)$, the $E.I.(t_k)$ is replaced with $E.I.(t_k) \times G(t_m)/E.I.(t_m)$. Then the radiation exposure dose corresponding to the contrast agent frame may be obtained based on the replaced $E.I.(t_k)$.

Further, a radiation exposure dose calculated using the E.I. based on the radiation image signal of the frame immediately preceding or immediately following the contrast agent frame may be employed directly as the radiation exposure dose of the patient during the capturing of the contrast agent frame or a radiation exposure dose calculated using the E.I. based on the radiation image signal of a frame other than the contrast agent frame may be employed. Otherwise, the radiation exposure dose calculated using the E.I. based on the radiation image signal of the contrast agent frame may be corrected by adding a predetermined radiation exposure dose determined in advance or the radiation exposure dose calculated based on the radiation image signal of the contrast agent frame may be corrected by multiplying it with a preset constant which is greater than 1.

Further, as for the contrast agent frame identification method, a method in which a photocounting radiation image detector is used as the radiation image detector 12 may be cited other than the aforementioned method. The photocounting radiation image detector is capable of counting the number of photons incident on the radiation image detector with respect to each of a plurality of energy bands. As such a photocounting radiation image detector is well known as described, for example, in Japanese Unexamined Patent Publication No. 2011-24773, it will not be elaborated upon further here.

Then, the main radiation absorption energy band of the contrast agent is set as one of the radiation energy bands which may be discriminated in the photocounting radiation image detector, then the state of decreasing in the number of photons counted in the energy band is monitored in the contrast agent frame identification unit, and a contrast agent frame may be identified by identifying a frame in which the number of photons is decreased to less than a predetermined threshold value. Note that the radiation energy band which may be discriminated in the photocounting radiation image detector may be arbitrarily set on the side of the radiation image detector. As for the method of obtaining the radiation exposure dose based on the signal detected by the radiation image detector is identical to that described above.

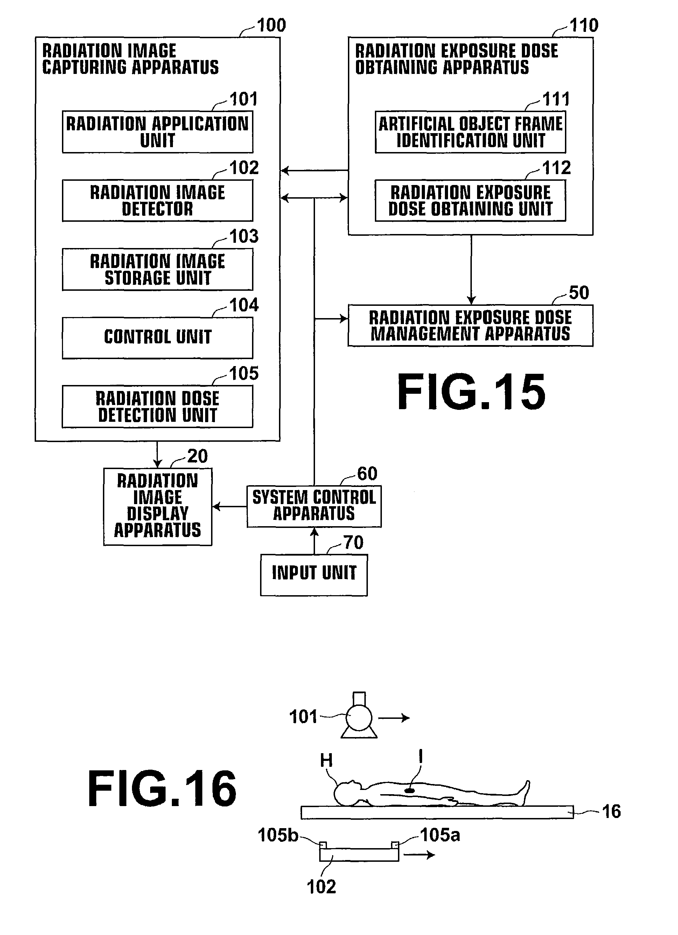

Next, a radiation image capturing system that uses a fourth embodiment of the radiation exposure dose obtaining apparatus of the present invention will be described. Figure 15 is a block diagram of the radiation image capturing system of the present embodiment, illustrating an overall schematic configuration thereof.

Whereas the radiation image capturing systems of the first to third embodiments perform radiological imaging of a patient injected with a contrast agent, the radiation image capturing system of the present invention performs radiological imaging of a patient with an embedded artificial object, such as an artificial bone. In the case where an artificial object, such as an artificial bone, is embedded in the body of the subject, if the exposure dose of the patient is tried to be obtained based simply on the image signal of the radiation image detector, there arises a concern the calculated value may possibly differ from the actual exposure dose of the patient, as in the case where a contrast agent is injected. That is, the radiation is absorbed by the artificial object and the amount of radiation reached the radiation image detector is reduced.

Further, in the case where a fluoroscopic image is captured for a patient with an embedded artificial object while moving the application range of radiation, not all of the frames of the radiological imaging of the fluoroscopic image but some of them will include an image of the artificial object. Therefore, it is necessary to obtain the exposure dose of the patient throughout the radiological imaging of the fluoroscopic image by taking into account the point described above.

The radiation image capturing system of the present embodiment is formed in order to solve the aforementioned problem.

More specifically, as illustrated in Figure 15, the radiation image capturing apparatus of the present embodiment includes a radiation image capturing apparatus 100 that captures a fluoroscopic image (moving picture) of a patient while moving the radiation application range and the radiation image detector relative to the patient, a radiation image display apparatus 20 that displays the fluoroscopic image captured by the radiation image capturing apparatus 100, a radiation exposure dose obtaining apparatus 110 that obtains an exposure dose of the patient based on an image signal of the fluoroscopic image captured by the radiation image capturing apparatus 100, a radiation exposure dose management apparatus 50 that stores and manages the exposure dose of each patient obtained by the radiation exposure dose obtaining apparatus 110, a system control apparatus 60 that performs overall control of the radiation image capturing system, and an input unit 70.

As illustrated in Figure 15, the radiation image capturing apparatus 100 includes a radiation application unit 101 that has an X-ray tube, an aperture stop, and the like and applies radiation emitted from the x-ray tube and passed through the aperture stop to a patient, a radiation image detector 102 that detects radiation transmitted through the patient and outputs a radiation image signal representing a radiation image of the patient, a radiation image storage unit 103 that stores the radiation image signal outputted from the radiation image detector 102, a control unit 104 that performs overall control of the radiation image capturing apparatus 100, and a radiation dose detection unit 105 provided between the patient and the radiation image detector 102 and detects the dose of radiation transmitted through the patient.

More specifically, the radiation image capturing apparatus 100 is structured as illustrated in Figure 16 and performs fluoroscopic image capturing by placing a patient H with an artificial object I (e.g., artificial bone) embedded in the body on the imaging platform 16 while moving the radiation application unit 101 and the radiation image detector 102 relative to the patient in the arrow direction shown in Figure 2.

The structure of the radiation image detector 102 is identical to that of the radiation image detector 12 of the first embodiment described above.

The radiation image capturing apparatus 100 is used to capture a fluoroscopic image (moving picture) of a patient with an embedded artificial object as described above, and the control unit 104 controls the radiation application unit 101 and the radiation image detector 102 such that the fluoroscopic image is captured. More specifically, the control unit 104 controls the radiation application unit 101 to apply radiation at a predetermined frame rate and the radiation image detector 102 to perform recording and reading of a radiation image by the application of the radiation. Then, the radiation image signal of each frame outputted from the radiation image detector 102 is sequentially stored in the radiation image storage unit 103.

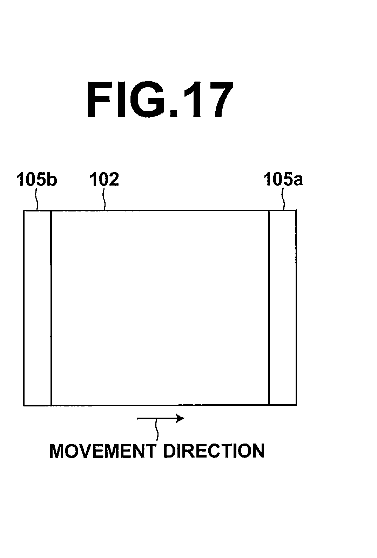

The radiation dose detection unit 105 is provided between the patient and the radiation image detector 102, and is formed of a first dose measurement sensor 105a and a second dose measurement sensor 105b provided on the radiation receiving surface of the radiation image detector 102 in the present embodiment, as illustrated in Figures 16 and 17. Figure 17 is a view of the radiation image detector 102 and the first and second dose measurement sensors 105a, 105b shown in Figure 16 viewed from above.

As illustrated in Figure 17, the first dose measurement sensor 105a and the second measurement sensor 105b are provided along the opposite sides in the movement direction of the radiation image detector 102. In the present embodiment, dose measurement sensors are provided along only the opposite sides in the movement direction as described above, but it is more preferable that dose measurement sensors are provided along the four sides of the radiation image detector 102.

As for the first and second dose measurement sensors 105a, 105b, the use of a thin sensor with substantially no radiation absorption is preferable and, for example, a sensor made of an organic photoelectric conversion material (OPC) is preferably used.

In the present embodiment, the first and second dose measurement sensors 105a, 105b are provided on the radiation image detector 102, but not limited to this and they may be set at the other predetermined position as long as it is between the patient and radiation image detector 102.

The radiation dose detection unit 105 sequentially outputs information of the detected dose of radiation to an artificial object frame identification unit 111, to be described later, in parallel with fluoroscopic image capturing.

As for the structure of the radiation image capturing apparatus 100, a structure in which image capturing is performed with the patient being in the lateral position, as shown in Figure 16, or in the upright position may be employed. Further, a structure that allows image capturing with the patient being both in upright position and lateral position may be employed.

The radiation exposure dose obtaining apparatus 110 includes an artificial object frame identification unit 111 that identifies a radiation image signal of a frame which includes an image signal of an image of an artificial object as an artificial object frame from the radiation image signal of each frame read out from the radiation image storage unit 103 of the radiation image capturing apparatus 100, and a radiation exposure dose obtaining unit 112 that obtains a radiation exposure dose of the patient based on the radiation image signal of each frame read out from the radiation image storage unit 103 of the radiation image capturing apparatus 100.

The artificial object frame identification unit 111 identifies an artificial object frame from a plurality of frames obtained by the fluoroscopic image capturing based on information of radiation dose outputted from the first dose measurement sensor 105a and the second dose measurement sensor 105b constituting the radiation dose detection unit 105.

Further, the artificial object frame identification unit 111 stores the radiation image signal of a frame determined to be an artificial object frame after appending information indicating as being an artificial object frame.

Operations of the artificial object frame identification unit 111 and the radiation exposure dose obtaining unit 112 will be described later in detail.

The radiation image display apparatus 20, the radiation exposure dose management apparatus 50, the system control apparatus 60, and input unit 70 are identical to those of the first embodiment.

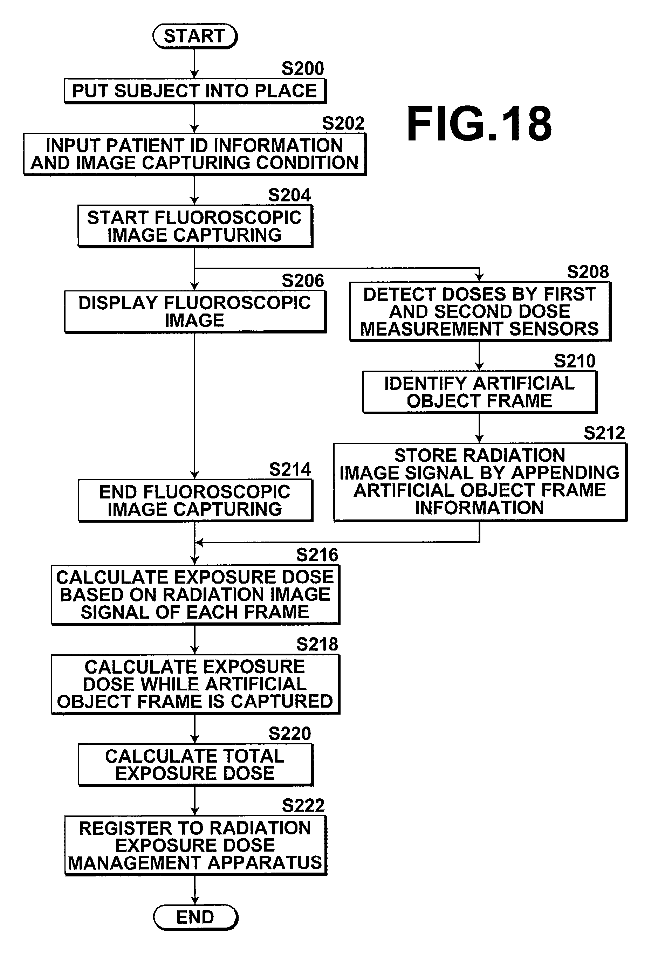

An operation of the radiation image capturing system of the present embodiment will now be described with reference to the flowchart of Figure 18.

First, a patient, the subject of the image capturing, is placed on an image capturing platform 16 provided in the radiation image capturing apparatus 100 and the patient is put into position (S200).

Then, ID information of the image capturing target patient and a given image capturing condition are inputted by the user using the input unit 70 and the patient ID information is registered in the radiation exposure dose management apparatus 50 while the image capturing condition is set to the control unit 104 of the radiation image capturing apparatus 100 (S202). The image capturing condition may include a tube voltage, tube current, and application time in order to apply an appropriate dose of radiation to an image capturing region of the patient, as well as a frame rate for capturing a fluoroscopic image. As for the frame rate for capturing the fluoroscopic image, for example, a frame rate of 5fps to 60fps is set.

Next, an instruction to start fluoroscopic image capturing of the patient is inputted by the user using the input unit 70 and in response to the input, a control signal is outputted from the system control apparatus 60 to the radiation image capturing apparatus 100 to capture a fluoroscopic image, and the radiation image capturing apparatus 100 starts fluoroscopic image capturing according to the inputted control signal (S204).

More specifically, the radiation application unit 101 and the radiation image detector 102 are moved relative to the patient in response to the inputted control signal and the X-ray tube of the radiation application unit 101 is controlled based on the inputted imaging condition and a predetermined dose of radiation is applied toward the patient intermittently at a predetermined frame rate.

Then, radiation transmitted through the patient is applied to the radiation image detector 102 and subjected to a photoelectrical conversion in the radiation image detector 102 and accumulated therein as a charge signal.

Thereafter, each time the application of radiation for each frame is ended, the charge signal accumulated in the radiation image detector 12 is read out by the control unit 104, then converted to a digital signal by an A/D converter (not shown), and stored in the radiation image storage unit 103. Note that the application timing of radiation from the X-ray tube of the radiation application unit 101 and the charge accumulation timing of the radiation image detector 102 are as in the timing chart of the first embodiment shown in Figure 4.

The repeated performance of radiation application by the X-ray tube and the radiation image recording and reading in the radiation image detector 102 at a predetermined frame rate in the manner described above causes the radiation image signal of each frame to be sequentially stored in the radiation image storage unit 103.

Then, the radiation image signal of each frame stored in the radiation image storage apparatus 103 is sequentially read out and outputted to the radiation image display apparatus 20. The radiation image display apparatus 20 sequentially generates a display control signal based on the inputted radiation image signal of each frame and sequentially outputs the display control signal to the monitor to display a fluoroscopic image of the patient as a moving picture (S206).

In the mean time, the dose of radiation transmitted through the patient is sequentially detected by the first and second dose measurement sensors 105a, 105b provided on the radiation image detector 102 in parallel with the fluoroscopic image capturing and display described above, and the detected dose of radiation is sequentially inputted to the artificial object frame identification unit 111 of the radiation exposure dose obtaining apparatus 110 (S208).

Then, the artificial object frame identification unit 111 obtains a temporal variation in the inputted dose of radiation, and identifies an artificial object frame in which an image of an artificial object is imaged based on the temporal variation (S210).

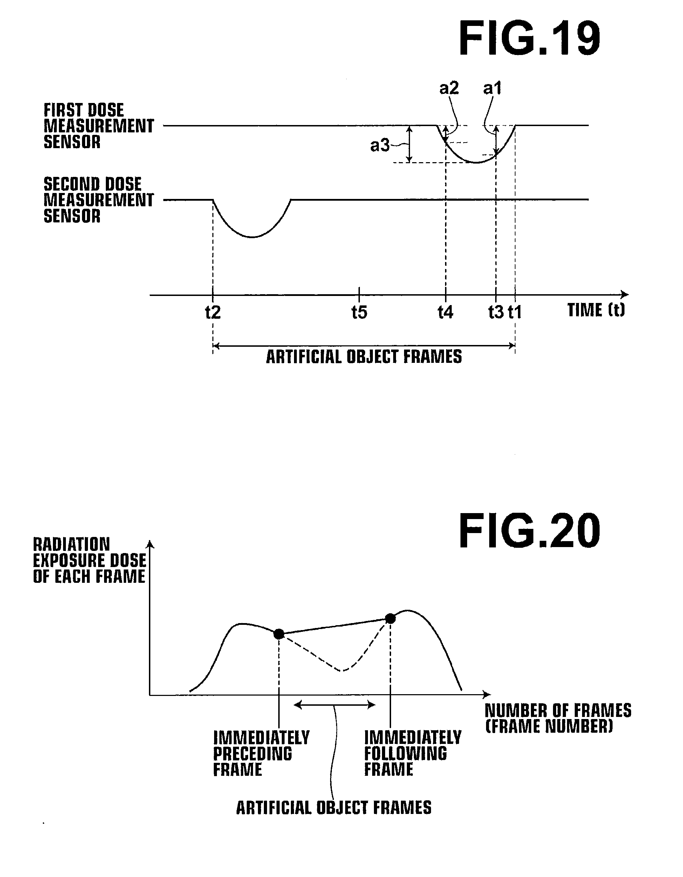

More specifically, the artificial object frame identification unit 111 obtains, based on the dose of radiation detected by the first dose measurement sensor 105a and the dose of radiation detected by the second dose measurement sensor 105b, a temporal variation in the dose of radiation detected by each sensor, as illustrated in Figure 19.

Here, when an artificial object embedded in the patient passes over the first and second dose measurement sensors 105a, 105b, the radiation is absorbed by the artificial object and the dose of radiation detected by each sensor is reduced, as illustrated in Figure 19.

By using such a change, the artificial object frame identification unit 111 identifies a frame captured from a first time point t1 at which the dose of radiation detected by the first dose measurement sensor 105a disposed in the downstream begins to decrease to a time point t2 at which the dose of radiation detected by the second dose measurement sensor 105b disposed in the upstream once decreased and then returned to a substantially constant value as an artificial object frame. It is assumed here that the movement speed of the radiation application unit 101 and the radiation image detector 102 and the frame rate of radiological imaging of the fluoroscopic image are preset in the artificial object frame identification unit 111, and the artificial object frame identification unit 111 identifies an artificial object frame based on the information of these and the time from the first time point t1 to the second time point t2.

The radiation image signal read out from the radiation image storage unit 103 is inputted also to the artificial object frame identification unit 111, and the artificial object frame identification unit 111 appends information indicating as being an artificial object frame (hereinafter, referred to as "artificial object frame information") to the radiation image signal of the frame identified as an artificial object frame in the manner described above as header information and stores the radiation image signal with the artificial object frame information (S212).

Thereafter, when an instruction to end the fluoroscopic image capturing is inputted by the user using the input unit 70, the system control apparatus 60 outputs a control signal to the radiation image capturing apparatus 100 to end the fluoroscopic image capturing, and the radiation image capturing apparatus 100 ends the fluoroscopic image capturing in response to the inputted control signal (S214).

When the fluoroscopic image capturing described above is ended, a radiation exposure dose of the patient is obtained in the radiation exposure dose obtaining apparatus 110.

More specifically, the radiation image signal of each frame stored in the artificial object frame identification unit 111 is outputted to the radiation exposure dose obtaining unit 112 with the artificial object frame information.

Next, a radiation exposure dose received by the patient during imaging of each frame of the fluoroscopic image is calculated in the radiation exposure dose obtaining unit 112 based on the radiation image signal of each frame (S216). More specifically, an E.I. is calculated based on the radiation image signal of each frame and a radiation exposure dose is obtained based on the E.I. also in the present embodiment. The calculation method of E.I. and radiation exposure dose obtaining method base on the E.I. are as explained in the first embodiment.

Here, if a fluoroscopic image of a patient with an embedded artificial object is captured, the radiation applied to the patient is absorbed by the artificial object before reaching the radiation image detector 102. Therefore, if a radiation exposure dose is calculated based on the radiation image signal of the artificial object frame, the calculated value will become smaller than that actually received by the patient.

Consequently, in the present embodiment, actual radiation exposure doses of the patient during the capturing of artificial object frames are obtained by obtaining radiation exposure doses corrected based on the information of artificial object frames identified in the artificial object frame identification unit 111 (S218).

More specifically, the radiation exposure dose obtaining unit 112 corrects the radiation exposure dose calculated based on the radiation image signal of the artificial object frame by adding a predetermined correction exposure dose. In the present embodiment, dose information detected by the first or second dose measurement sensor 105a or 105b during the correction target artificial object frame is captured as the correction exposure dose. That is, the decrease in the detected dose by each sensor due to radiation absorption by the artificial object is used as the correction exposure dose. For example, the radiation exposure dose corresponding to the artificial object frame captured at the time point t3 in Figure 19 is corrected by adding the correction exposure dose a1. The radiation exposure dose corresponding to the artificial object frame captured at the time point t4 in Figure 19 is corrected by adding the correction exposure dose a2. For radiation doses corresponding to artificial object frames captured while the artificial object is passing between the first dose measurement sensor 105a and the second dose measurement sensor 105b, a maximum correction dose a3 when the detection dose of each sensor is decreased the most is added. That is, for example, the radiation exposure dose corresponding to the artificial object frame captured at the time point t5 in Figure 19 is corrected by adding the correction exposure dose a3.

Next, the radiation exposure dose obtaining unit 112 calculates the total radiation exposure dose received by the patient by adding the radiation exposure doses during the capturing of the artificial object frames obtained in the manner described above to the radiation exposure doses during the capturing of frames other than the artificial object frames (S220).

The total radiation exposure dose obtained in the radiation exposure dose obtaining unit 112 in the manner described above is inputted to the radiation exposure dose management apparatus 50, and the radiation exposure dose management apparatus 50 registers the total radiation exposure dose with the ID information of the patient inputted in advance (S222). Then, the radiation exposure dose management apparatus 50 displays the registered total radiation exposure dose with the ID information of the patient as required or calculates a cumulative radiation exposure dose from the past for a particular patient and displays a warning message if the cumulative radiation exposure dose is greater than a predetermined specified value.

According to the radiation image capturing system of the present embodiment, the radiation exposure dose of a living body is obtained for the radiological imaging of an artificial object by identifying a frame which includes an image signal representing an image of an artificial object as an artificial object frame from a plurality of frames based on dose information of radiation detected by the radiation dose detection unit 105 provided between the patient and radiation image detector 102, and correcting the radiation exposure dose based on the information of the identified artificial object frame. This allows appropriate identification of an artificial object frame to be made by a simple structure. Further, a radiation exposure dose throughout the radiological imaging which takes into account the inclusion of an image of an artificial object in the radiation image may be obtained more accurately.

Further, the radiation exposure dose is obtained for the radiological imaging of an artificial object by correcting the radiation exposure dose obtained base on the radiation image signal of the artificial object frame using the dose information of radiation detected by the radiation dose detection unit 105, so that an accurate radiation exposure dose may be obtained by a simpler correction method.

In the radiation image capturing system of the fourth embodiment described above, the radiation exposure dose is calculated after the fluoroscopic image capturing is completed, but not limited to this and the radiation exposure dose is calculated in the manner described above in the middle of the fluoroscopic image capturing and the radiation exposure dose of a subject in the middle of the imaging may be displayed or the like.

Further, in the radiation image capturing system of the fourth embodiment described above, the correction is performed by adding a correction exposure dose to the radiation exposure dose calculated based on the radiation image signal of the artificial object frame, but the method for obtaining a radiation exposure dose during imaging of an artificial object is not limited to this and, for example, a radiation exposure dose of the patient during imaging of an artificial object may be obtained through linear interpolation of radiation exposure doses calculated based on the frames immediately preceding and immediately following the artificial object frame, as illustrated in Figure 20.

Further, a radiation exposure dose calculated based on the radiation image signal of the frame immediately preceding or immediately following the artificial object frame may be employed directly as the radiation exposure dose during the artificial object frame capturing, or a radiation exposure dose calculated based on the radiation image signal of another frame other than the artificial object frame may be employed. Otherwise, a radiation exposure dose calculated based on the radiation image signal of an artificial object frame may be corrected by adding a predetermined radiation exposure dose determined in advance or by multiplying it with a given coefficient which is greater than 1.

Still further, in the radiation image capturing system of the fourth embodiment described above, an artificial object frame is identified based on the temporal variations in the doses of radiation detected by the first and second dose measurement sensors 105a, 105b, but not limited to this and, for example, an index, such as a mark, indicating that it is an artificial object may be provided on an artificial object to be embedded in the body of a patient, and an artificial object frame may be identified by the artificial object frame identification unit 111 through image recognition as to whether or not an image signal representing the aforementioned index is included in the radiation image signal of each frame. If such arrangement is adopted, an artificial object may be identified only by performing image recognition, so that the artificial object frame may be identified by a simpler structure. As the mark image recognition is an already known technique, it will not be elaborated upon further here.

In the case where an index is provided for an artificial object as described above, the index may include not only the information that indicates that it is an artificial object but also information related to correction of the radiation exposure dose. Then, a radiation exposure dose corresponding to the artificial object frame may be corrected by the radiation exposure dose obtaining unit 112 by obtaining the information related to the correction included in the index. More specifically, the index may include, for example, information related to absorption of radiation, such as the material, thickness, and shape of the artificial object while a correction radiation exposure dose corresponding to the aforementioned information is set in the radiation exposure dose obtaining unit 112 in advance. Then, a correction may be performed by the radiation exposure dose obtaining unit 112 by adding the correction radiation exposure dose corresponding to the obtained information described above to the radiation exposure dose calculated based on the radiation image signal of the artificial object frame. If such arrangement is adopted, the correction may be performed easier without requiring, in particular, complicated calculations.

Also, the artificial object frame identification method may include a method that uses a photocounting radiation image detector, as in the contrast agent frame described above. In this case, the main radiation absorption energy band of the artificial object may be set as one of the radiation energy bands which may be discriminated in the photocounting radiation image detector and an artificial object may be identified by monitoring the state of decreasing in the number of photons counted in the energy band in the artificial object frame identification unit.

Further, in the first to fourth embodiments, the radiation exposure dose obtaining apparatus is implemented as an independent apparatus, but it may be implemented in any other form, such as being incorporated in the other apparatus, such as the radiation image capturing apparatus, as a part thereof. More specifically, it may be provided in a console which includes the system control apparatus 60 or in the radiation image capturing apparatus 10 or 100. Further, in the case where the radiation image detector 12 or 102 is accommodated in a portable electronic cassette, and hardware of electronic circuits, such as LSI (Large Scale Integration), hardware of programmable electronic circuits, such as PLD (Programmable Logic Device) · FPGA (Field-Programmable Gate Array), and the like are accommodated in the electronic cassette, the identification of an artificial object frame and acquisition of the radiation exposure dose may be performed by such hardware. Such arrangement allows pursuit of more real time implementation.

**Claims**

1. A radiation exposure dose obtaining apparatus, comprising:

    a radiation exposure dose obtaining unit that obtains, based on a radiation image signal of each frame detected by a radiation image detector through continuous radiological imaging of a subject to be injected with a contrast agent, a radiation exposure dose of the subject due to the continuous radiological imaging; and
    a contrast agent frame identification unit that identifies a frame which includes an image signal representing an image of the contrast agent as a contrast agent frame from the radiation image signal of each frame, wherein the radiation exposure dose obtaining unit corrects the radiation exposure dose based on information of the contrast agent frame and obtains a radiation exposure dose of the subject for the radiological imaging of the contrast agent frame.

2. The radiation exposure dose obtaining apparatus of claim 1, wherein the contrast agent frame identification unit obtains information that indicates whether or not the contrast agent is being injected into the subject and identifies the contrast agent frame based on the obtained information.

3. The radiation exposure dose obtaining apparatus of claim 2, wherein the contrast agent frame identification unit obtains the information that indicates whether or not the contrast agent is being injected into the subject by obtaining information appended to the radiation image signal of each frame.

4. The radiation exposure dose obtaining apparatus of claim 3, wherein the information appended to the radiation image signal of each frame is header information of the radiation image signal.

5. The radiation exposure dose obtaining apparatus of any of claims 2 to 4, wherein the information that indicates whether or not the contrast agent is being injected into the subject is based on a detection signal detected by a sensor provided in a contrast agent injection apparatus that injects the contrast agent into the subject.

6. The radiation exposure dose obtaining apparatus of claim 2, wherein the information that indicates whether or not the contrast agent is being injected into the subject is information that indicates an injection start timing and an injection end timing of the contrast agent.

7. The radiation exposure dose obtaining apparatus of claim 6, wherein the information that indicates an injection start timing and an injection end timing of the contrast agent are an injection start time and an injection end time of the contrast agent into the subject.

8. The radiation exposure dose obtaining apparatus of claim 7, wherein the injection start time is a drive start time of a contrast agent injection apparatus that injects the contrast agent into the subject and the injection end time is a drive end time of the contrast agent injection apparatus.

9. A radiation image capturing system, comprising:

the radiation exposure dose obtaining apparatus of claim 3; and
a radiation image capturing apparatus that obtains the radiation image signal of each frame by performing the radiological imaging,
wherein the radiation image capturing apparatus obtains information that indicates whether or not the contrast agent is being injected into the subject and stores the radiation image signal after appending the information to the radiation image signal.

10. The radiation image capturing system of claim 9, comprising a contrast agent injection apparatus that injects the contrast agent to the subject,
wherein the radiation image capturing apparatus obtains the information based on a detection signal detected by a sensor provided in the contrast agent injection apparatus.

11. The radiation exposure dose obtaining apparatus of claim 1, wherein the contrast agent frame identification unit identifies the contrast agent frame based on difference information which is based on radiation image signals between predetermined frames of those of a plurality of frames detected through the continuous radiological imaging.

12. The radiation exposure dose obtaining apparatus of claim 11, wherein the contrast agent frame identification unit identifies the contrast agent frame based on a difference between a density histogram based on the radiation image signal of an $n^{th}$ frame (n is an integer greater than or equal to 2) of the radiation image signals of the plurality of frames and a density histogram based on the radiation image signal of a $(n-1)^{th}$ frame.

13. The radiation exposure dose obtaining apparatus of claim 11, wherein the contrast agent frame identification unit identifies the contrast agent frame based on a difference in each corresponding pixel value between the radiation image signal of an $n^{th}$ frame (n is an integer greater than or equal to 2) of the radiation image signals of the plurality of frames and the radiation image signal of a $(n-1)^{th}$ frame.

14. The radiation exposure dose obtaining apparatus of claim 1, wherein the contrast agent frame identification unit obtains dose information of radiation outputted from a radiation dose detection unit provided between a radiation source that emits the radiation to be applied to the subject and the subject, and identifies the contrast agent frame based on the obtained dose information and the radiation image signals of a plurality of frames detected through the continuous radiological imaging.

15. The radiation exposure dose obtaining apparatus of claim 14, wherein the contrast agent frame identification unit obtains a temporal variation in the dose information while the continuous radiological imaging is performed.

16. The radiation exposure dose obtaining apparatus of claim 14 or 15, wherein the contrast agent frame identification unit calculates an index of dose of radiation received by the radiation image detector based on the radiation image signals of a plurality of frames detected through the continuous radiological imaging and obtains a temporal variation in the index while the continuous radiological imaging is performed.

17. The radiation exposure dose obtaining apparatus of claim 16, wherein the contrast agent frame identification unit calculates a ratio between the dose information of radiation and the index calculated based on the radiation image signal of each frame, and identifies the contrast agent frame based on the ratio.

18. The radiation exposure dose obtaining apparatus of claim 17, wherein the radiation exposure dose obtaining unit obtains a radiation exposure dose of the subject for the radiological imaging of the contrast agent frame based on the index calculated based on the radiation image signal of the contrast agent frame and a ratio between the dose information of radiation detected in the radiological imaging other than the contrast agent frame and the index calculated based on the radiation image signal other than the contrast agent frame.

19. The radiation exposure dose obtaining apparatus of any of claims 14 to 18, wherein the radiation dose detection unit is an area dosimeter that measures the radiation emitted from the radiation source.

20. The radiation exposure dose obtaining apparatus of any of claims 11 to 19, the contrast agent frame identification unit appends information indicating as being a contrast agent frame to the frame identified as a contrast agent frame.

21. The radiation exposure dose obtaining apparatus of claim 20, wherein the information indicating as being a contrast agent frame is header information of the radiation image signal.

22. A radiation exposure dose obtaining method that obtains, based on a radiation image signal of each frame detected by a radiation image detector through continuous radiological imaging of a subject to be injected with a contrast agent, a radiation exposure dose of the subject due to the continuous radiological imaging, the method comprising the steps of:

identifying a frame which includes an image signal representing an image of the contrast agent as a contrast agent frame from the radiation image signal of each frame; and
correcting the radiation exposure dose based on information of the contrast agent frame and obtaining a radiation exposure dose of the subject for the radiological imaging of the contrast agent frame.

23. The radiation exposure dose obtaining method of claim 22, wherein information indicating whether or not the contrast agent is being injected into the subject is obtained, and the contrast agent frame is identified based on the obtained information.

24. The radiation exposure dose obtaining method of claim 22, wherein the contrast agent frame is identified based on difference information which is based on radiation image signals between predetermined frames of those of a plurality of frames detected through the continuous radiological imaging.

25. The radiation exposure dose obtaining method of claim 22, wherein dose information of radiation outputted from a radiation dose detection unit provided between a radiation source that emits the radiation to be applied to the subject and the subject, and the contrast agent frame is identified based on the obtained dose information and the radiation image signals of a plurality of frames detected through the continuous radiological imaging.

## RADIATION IMAGE CAPTURING APPARATUS — 10

### RADIATION APPLICATION UNIT — 11

### RADIATION IMAGE DETECTOR — 12

### RADIATION IMAGE STORAGE UNIT — 13

### CONTROL UNIT — 14

## CONTRAST AGENT INJECTION APPARATUS — 30

### CONTRAST AGENT INJECTION UNIT — 31

### INJECTION DRIVE UNIT — 32

### CONTROL UNIT — 33

### CONTRAST AGENT INJECTION DETECTION SENSOR UNIT — 34

## RADIATION IMAGE DISPLAY APPARATUS — 20

## RADIATION EXPOSURE DOSE OBTAINING APPARATUS — 40

### CONTRAST AGENT FRAME IDENTIFICATION UNIT — 41

### RADIATION EXPOSURE DOSE OBTAINING UNIT — 42

## SYSTEM CONTROL APPARATUS — 60

## INPUT UNIT — 70

## RADIATION EXPOSURE DOSE MANAGEMENT APPARATUS — 50

# FIG.1

# FIG.2

31a 31b 32a 32b — M 32c

PM 32d

## SYSTEM CONTROL APPARATUS — 60

## CONTROL UNIT — 33

# FIG.3

(START)

↓ S10
PUT SUBJECT INTO PLACE

↓ S12
INPUT PATIENT ID INFORMATION AND IMAGE CAPTURING CONDITION

↓ S14
START FLUOROSCOPIC IMAGE CAPTURING

↓ S16
DISPLAY FLUOROSCOPIC IMAGE

↓ S18
INPUT CONTRAST AGENT INJECTION INSTRUCTION

↓ S20
DETECT THAT CONTRAST AGENT IS BEING INJECTED

↓ S22
STORE RADIATION IMAGE SIGNAL BY APPENDING CONTRAST AGENT FRAME INFORMATION

↓ S24
DISPLAY CONTRAST AGENT IMAGE

↓ S26
END FLUOROSCOPIC IMAGE CAPTURING

↓ S28
IDENTIFY CONTRAST AGENT FRAME

↓ S30
CALCULATE EXPOSURE DOSE BASED ON RADIATION IMAGE SIGNAL OF EACH FRAME

↓ S32
CALCULATE EXPOSURE DOSE WHILE CONTRAST AGENT IS INJECTED

↓ S34
CALCULATE TOTAL EXPOSURE DOSE

↓ S36
REGISTER TO RADIATION EXPOSURE DOSE MANAGEMENT APPARATUS

↓
(END)

**FRAME**

X-RAY OUTPUT
OF X-RAY TUBE

ON

OFF

CONTRAST
AGENT
DETECTION
SIGNAL

BEING
INJECTED

BEING NOT
INJECTED

CHARGE
ACCUMULATION
OF RADIATION
IMAGE DETECTOR

ACCUMULATION
ON

ACCUMULATION
OFF

F1  F2

RADIATION EXPOSURE
DOSE OF EACH FRAME

# FIG.4

F1  F2

NUMBER OF FRAMES
(FRAME NUMBER)

IMMEDIATELY
PRECEDING
FRAME

IMMEDIATELY
FOLLOWING
FRAME

**RADIATION IMAGE CAPTURING APPARATUS** ⌐10

**RADIATION APPLICATION UNIT** ⌐11

**RADIATION IMAGE DETECTOR** ⌐12

**RADIATION IMAGE STORAGE UNIT** ⌐13

**CONTROL UNIT** ⌐14

**CONTRAST AGENT INJECTION APPARATUS** ⌐30

**CONTRAST AGENT INJECTION UNIT** ⌐31

**INJECTION DRIVE UNIT** ⌐32

**CONTROL UNIT** ⌐33

**CONTRAST AGENT INJECTION TIME OBTAINING UNIT** ⌐35

**RADIATION EXPOSURE DOSE OBTAINING APPARATUS** ⌐40

**CONTRAST AGENT FRAME IDENTIFICATION UNIT** ⌐41

**RADIATION EXPOSURE DOSE OBTAINING UNIT** ⌐42

**RADIATION IMAGE DISPLAY APPARATUS** ⌐20

**SYSTEM CONTROL APPARATUS** ⌐60

**INPUT UNIT** ⌐70

**RADIATION EXPOSURE DOSE MANAGEMENT APPARATUS** ⌐50

# FIG.5

RADIATION IMAGE
CAPTURING APPARATUS — 10

RADIATION APPLICATION
UNIT — 11

RADIATION IMAGE
DETECTOR — 12

RADIATION IMAGE
STORAGE UNIT — 13

CONTROL UNIT — 14

CONTRAST AGENT
INJECTION APPARATUS — 30

CONTRAST AGENT
INJECTION UNIT — 31

INJECTION DRIVE UNIT — 32

CONTROL UNIT — 33

RADIATION
IMAGE
DISPLAY
APPARATUS — 20

RADIATION EXPOSURE DOSE
OBTAINING APPARATUS — 80

CONTRAST AGENT FRAME
IDENTIFICATION UNIT — 81

RADIATION EXPOSURE DOSE
OBTAINING UNIT — 82

SYSTEM CONTROL
APPARATUS — 60

RADIATION EXPOSURE DOSE
MANAGEMENT APPARATUS — 50

INPUT UNIT — 70

# FIG.6

**FIG.7**

```
              ( START )
                  │
                  ▼                    S40
        ┌────────────────────┐
        │ PUT SUBJECT INTO PLACE │
        └────────────────────┘
                  │
                  ▼                    S42
        ┌──────────────────────────┐
        │ INPUT PATIENT ID INFORMATION │
        │ AND IMAGE CAPTURING CONDITION │
        └──────────────────────────┘
                  │
                  ▼                    S44
        ┌────────────────────┐
        │ START FLUOROSCOPIC   │
        │ IMAGE CAPTURING      │
        └────────────────────┘
                  │
                  ▼                    S46
        ┌────────────────────────┐
        │ DISPLAY FLUOROSCOPIC IMAGE │
        └────────────────────────┘
                  │
        ┌─────────┴──────────────────────────┐
        ▼               S48                   ▼               S52
┌────────────────────┐         ┌────────────────────┐
│ INPUT CONTRAST AGENT │         │ IDENTIFY CONTRAST    │
│ INJECTION INSTRUCTION │         │ AGENT FRAME          │
└────────────────────┘         └────────────────────┘
        │               S50                   │               S54
        ▼                                     ▼
┌────────────────────┐         ┌────────────────────────┐
│ DISPLAY CONTRAST     │         │ STORE RADIATION IMAGE    │
│ AGENT IMAGE          │         │ SIGNAL BY APPENDING      │
└────────────────────┘         │ CONTRAST AGENT           │
        │               S56     │ FRAME INFORMATION        │
        ▼                       └────────────────────────┘
┌────────────────────┐                       │
│ END FLUOROSCOPIC     │                       │
│ IMAGE CAPTURING      │                       │
└────────────────────┘                       │
        │◄──────────────────────────────────────┘
        ▼               S58
┌────────────────────────┐
│ CALCULATE EXPOSURE DOSE   │
│ BASED ON RADIATION IMAGE  │
│ SIGNAL OF EACH FRAME      │
└────────────────────────┘
        │               S60
        ▼
┌────────────────────────┐
│ CALCULATE EXPOSURE DOSE   │
│ WHILE CONTRAST AGENT      │
│ IS INJECTED               │
└────────────────────────┘
        │               S62
        ▼
┌────────────────────────────┐
│ CALCULATE TOTAL EXPOSURE DOSE │
└────────────────────────────┘
        │               S64
        ▼
┌────────────────────────────┐
│ REGISTER TO RADIATION EXPOSURE │
│ DOSE MANAGEMENT APPARATUS      │
└────────────────────────────┘
        │
        ▼
     (  END  )
```

# FIG.8

FREQUENCY

DENSITY

WHITE          BLACK

HISTOGRAM OF nth FRAME
(WITH CONTRAST AGENT)

FREQUENCY

DENSITY

WHITE          BLACK

HISTOGRAM OF (n-1)th FRAME
(WITHOUT CONTRAST AGENT)

FREQUENCY

THRESHOLD VALUE

0          DENSITY

THRESHOLD VALUE

WHITE          BLACK

SUBTRACTION HISTOGRAM

EP 2 716 222 A1

# FIG.9

CONTRAST AGENT INJECTION START FRAME

f1 → f2 → f3 → f4 → f5 →

f6 → f7 → f8 → f9 → f10

CONTRAST AGENT INJECTION END FRAME

# FIG.10

n$^{th}$ FRAME                     (n-1)$^{th}$ FRAME

QL(x, y, n)                     QL(x, y, n-1)

**FIG.11**

RADIATION IMAGE CAPTURING APPARATUS — 10

RADIATION APPLICATION UNIT — 11

RADIATION IMAGE DETECTOR — 12

RADIATION IMAGE STORAGE UNIT — 13

CONTROL UNIT — 14

RADIATION DOSE DETECTION UNIT — 15

CONTRAST AGENT INJECTION APPARATUS — 30

CONTRAST AGENT INJECTION UNIT — 31

INJECTION DRIVE UNIT — 32

CONTROL UNIT — 33

RADIATION IMAGE DISPLAY APPARATUS — 20

RADIATION EXPOSURE DOSE OBTAINING APPARATUS — 90

CONTRAST AGENT FRAME IDENTIFICATION UNIT — 91

RADIATION EXPOSURE DOSE OBTAINING UNIT — 92

SYSTEM CONTROL APPARATUS — 60

INPUT UNIT — 70

RADIATION EXPOSURE DOSE MANAGEMENT APPARATUS — 50

**FIG.12**

11
15
12

**FIG.13**

```
                    ( START )
                        │
                        ▼           S70
            ┌───────────────────────────┐
            │   PUT SUBJECT INTO PLACE   │
            └───────────────────────────┘
                        │
                        ▼                        S72
            ┌──────────────────────────────────────┐
            │    INPUT PATIENT ID INFORMATION       │
            │  AND IMAGE CAPTURING CONDITION        │
            └──────────────────────────────────────┘
                        │
                        ▼               S74
            ┌───────────────────────────┐
            │    START FLUOROSCOPIC      │
            │     IMAGE CAPTURING        │
            └───────────────────────────┘
                        │
                        ▼                   S76
            ┌───────────────────────────────────┐
            │   DISPLAY FLUOROSCOPIC IMAGE      │
            └───────────────────────────────────┘
                 │                              │
                 ▼          S78                 ▼          S82
      ┌──────────────────────┐      ┌──────────────────────────┐
      │ INPUT CONTRAST AGENT  │      │  DETECT DOSE BY RADIATION │
      │ INJECTION INSTRUCTION │      │   DOSE DETECTION UNIT     │
      └──────────────────────┘      └──────────────────────────┘
                 │          S80                 │          S84
                 ▼                              ▼
      ┌──────────────────────┐      ┌──────────────────────────┐
      │   DISPLAY CONTRAST    │      │   IDENTIFY CONTRAST       │
      │    AGENT IMAGE        │      │    AGENT FRAME            │
      └──────────────────────┘      └──────────────────────────┘
                 │          S88                 │          S86
                 ▼                              ▼
      ┌──────────────────────┐      ┌──────────────────────────┐
      │   END FLUOROSCOPIC    │      │ STORE E.I. BY APPENDING   │
      │   IMAGE CAPTURING     │      │ CONTRAST AGENT FRAME      │
      └──────────────────────┘      │      INFORMATION          │
                 │                  └──────────────────────────┘
                 ▼          S90
      ┌──────────────────────────┐
      │ CALCULATE EXPOSURE DOSE   │
      │ BASED ON RADIATION IMAGE  │
      │  SIGNAL OF EACH FRAME     │
      └──────────────────────────┘
                 │          S92
                 ▼
      ┌──────────────────────────────────┐
      │ CALCULATE EXPOSURE DOSE WHILE     │
      │  CONTRAST AGENT IS INJECTED       │
      └──────────────────────────────────┘
                 │          S94
                 ▼
      ┌──────────────────────────────────┐
      │ CALCULATE TOTAL EXPOSURE DOSE     │
      └──────────────────────────────────┘
                 │          S96
                 ▼
      ┌──────────────────────────────────┐
      │ REGISTER TO RADIATION EXPOSURE    │
      │ DOSE MANAGEMENT APPARATUS         │
      └──────────────────────────────────┘
                 │
                 ▼
            ( END )
```

# FIG.14

G(t)

G(t)/E.I.(t)

THRESHOLD VALUE

TIME (t)

TIME (t)

CONTRAST AGENT IS BEING INJECTED

E.I.(t)

TIME (t)

EP 2 716 222 A1

**FIG.15**

**FIG.16**

# FIG.17

105b    102                    105a

MOVEMENT DIRECTION

# FIG.18

START

S200
PUT SUBJECT INTO PLACE

S202
INPUT PATIENT ID INFORMATION
AND IMAGE CAPTURING CONDITION

S204
START FLUOROSCOPIC
IMAGE CAPTURING

S206
DISPLAY FLUOROSCOPIC
IMAGE

S208
DETECT DOSES BY FIRST
AND SECOND DOSE
MEASUREMENT SENSORS

S210
IDENTIFY ARTIFICIAL
OBJECT FRAME

S212
STORE RADIATION
IMAGE SIGNAL BY APPENDING
ARTIFICIAL OBJECT FRAME
INFORMATION

S214
END FLUOROSCOPIC
IMAGE CAPTURING

S216
CALCULATE EXPOSURE DOSE
BASED ON RADIATION IMAGE
SIGNAL OF EACH FRAME

S218
CALCULATE EXPOSURE
DOSE WHILE ARTIFICIAL
OBJECT FRAME IS CAPTURED

S220
CALCULATE TOTAL
EXPOSURE DOSE

S222
REGISTER TO RADIATION
EXPOSURE DOSE
MANAGEMENT APPARATUS

END

# FIG.19

FIRST DOSE
MEASUREMENT
SENSOR

a2  a1

a3

SECOND DOSE
MEASUREMENT
SENSOR

t2        t5        t4    t3 t1    TIME (t)

ARTIFICIAL OBJECT FRAMES

# FIG.20

RADIATION
EXPOSURE DOSE
OF EACH FRAME

NUMBER OF FRAMES
(FRAME NUMBER)

IMMEDIATELY
PRECEDING
FRAME

IMMEDIATELY
FOLLOWING
FRAME

ARTIFICIAL OBJECT FRAMES

## INTERNATIONAL SEARCH REPORT

| | International application No. |
| --- | --- |
| | PCT/JP2012/003459 |

**A. CLASSIFICATION OF SUBJECT MATTER**
*A61B6/00*(2006.01)i

According to International Patent Classification (IPC) or to both national classification and IPC

**B. FIELDS SEARCHED**

Minimum documentation searched (classification system followed by classification symbols)
A61B6/00

Documentation searched other than minimum documentation to the extent that such documents are included in the fields searched

| | | | |
| --- | --- | --- | --- |
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | JP 2010-5480 A (Toshiba Corp.), 14 January 2010 (14.01.2010), entire text; all drawings (Family: none) | 1-21 |
| A | JP 2005-270286 A (Toshiba Corp.), 06 October 2005 (06.10.2005), entire text; all drawings (Family: none) | 1-21 |
| A | JP 2011-92481 A (GE Medical Systems Global Technology Co., L.L.C.), 12 May 2011 (12.05.2011), entire text; all drawings (Family: none) | 1-21 |

☒ Further documents are listed in the continuation of Box C. ☐ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 24 August, 2012 (24.08.12) | 04 September, 2012 (04.09.12) |

| Name and mailing address of the ISA/ | Authorized officer |
| --- | --- |
| Japanese Patent Office | |
| Facsimile No. | Telephone No. |

Form PCT/ISA/210 (second sheet) (July 2009)

**INTERNATIONAL SEARCH REPORT**

| International application No. |
| --- |
| PCT/JP2012/003459 |

C (Continuation).    DOCUMENTS CONSIDERED TO BE RELEVANT

| Category* | Citation of document, with indication, where appropriate, of the relevant passages | Relevant to claim No. |
| --- | --- | --- |
| A | WO 2008/096813 A1  (Hitachi Medical Corp.), 14 August 2008 (14.08.2008), entire text; all drawings & US 2010/0104159 A1    & CN 101605498 A | 1-21 |

Form PCT/ISA/210 (continuation of second sheet) (July 2009)

## INTERNATIONAL SEARCH REPORT

| International application No. |
|---|
| PCT/JP2012/003459 |

**Box No. II      Observations where certain claims were found unsearchable (Continuation of item 2 of first sheet)**

This international search report has not been established in respect of certain claims under Article 17(2)(a) for the following reasons:

1. ☒  Claims Nos.: 22-25
   because they relate to subject matter not required to be searched by this Authority, namely:
   (See extra sheet)

2. ☐  Claims Nos.:
   because they relate to parts of the international application that do not comply with the prescribed requirements to such an extent that no meaningful international search can be carried out, specifically:

3. ☐  Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:

1. ☐  As all required additional search fees were timely paid by the applicant, this international search report covers all searchable claims.

2. ☐  As all searchable claims could be searched without effort justifying additional fees, this Authority did not invite payment of additional fees.

3. ☐  As only some of the required additional search fees were timely paid by the applicant, this international search report covers only those claims for which fees were paid, specifically claims Nos.:

4. ☐  No required additional search fees were timely paid by the applicant.   Consequently, this international search report is restricted to the invention first mentioned in the claims; it is covered by claims Nos.:

**Remark on Protest**      ☐  The additional search fees were accompanied by the applicant's protest and, where applicable, the payment of a protest fee.

☐  The additional search fees were accompanied by the applicant's protest but the applicable protest fee was not paid within the time limit specified in the invitation.

☐  No protest accompanied the payment of additional search fees.

Form PCT/ISA/210 (continuation of first sheet (2)) (July 2009)

| **INTERNATIONAL SEARCH REPORT** | International application No. |
| --- | --- |
| | PCT/JP2012/003459 |

<u>Continuation of Box No.II-1 of continuation of first sheet(2)</u>

Claims 22 to 25 relate to a method comprising "continuously radiographing a subject to whom a contrast medium is injected" and said method involves a step for conducting a surgical treatment on the human body, i.e., the injection of a contrast medium.

Therefore, claims 22-25 relate to a subject matter on which this International Searching Authority is not required to carry out a search under the provisions of PCT Article 17(2)(a)(i) and PCT Rule 39.1(iv).

Form PCT/ISA/210 (extra sheet) (July 2009)

**EP 2 716 222 A1**

## REFERENCES CITED IN THE DESCRIPTION

**Patent documents cited in the description**

- JP 4387644 B
- JP 2011024773 A